# EUROPÄISCHE PATENTSCHRIFT

(11) **EP 1 638 994 B1**
(45) Veröffentlichungstag und Bekanntmachung des Hinweises auf die Patenterteilung: **04.04.2012**
(21) Anmeldenummer: 04739975.3
(22) Anmeldetag: 17.06.2004
(51) Int. Cl.: C07K 14/02

(54) **NEUE OBERFLÄCHENPROTEIN- (HbsAg-) VARIANTE DES HEPATITIS B VIRUS**
NOVEL SURFACE PROTEIN (HBSAG) VARIANT OF HEPATITIS B VIRUS
NOUVELLE VARIANTE DE LA PROTEINE DE SURFACE (HBSAG-) DU VIRUS DE L'HEPATITE B

(30) Priorität: 20.06.2003 DE 10328080
(43) Veröffentlichungstag der Anmeldung: 29.03.2006
(73) Patentinhaber: Siemens Healthcare Diagnostics Products GmbH, 35041 Marburg (DE)
(72) Erfinder: KRUPKA, Udo, 35043 Marburg (DE)
(86) Internationale Anmeldenummer: PCT/EP2004/006515
(87) Internationale Veröffentlichungsnummer: WO 2004/113369

(56) Entgegenhaltungen:
- EP-A- 1 142 906
- EP-A- 1 174 523
- US-A- 5 531 990
- DEGEN S J F ET AL: "THE MURINE UROKINASE-TYPE PLASMINOGEN ACTIVATOR GENE" BIOCHEMISTRY, Bd. 26, Nr. 25, 1987, Seiten 8270-8279, XP002300765 ISSN: 0006-2960
- COOREMAN M P ET AL: "CHARACTERIZATION OF THE REACTIVITY PATTERN OF MURINE MONOCLONAL ANTIBODIES AGAINST WILD-TYPE HEPATITIS B SURFACE ANTIGEN TO G145R AND OTHER NATURALLY OCCURRING A LOOP ESCAPE MUTATIONS" HEPATOLOGY, WILLIAMS AND WILKINS, BALTIMORE, MD, US, Bd. 30, Nr. 6, November 1999 (1999-11), Seiten 1287-1292, XP001015475 ISSN: 0270-9139
- WEINBERGER KLAUS M ET AL: "High genetic variability of the group-specific a-determinant of hepatitis B virus surface antigen (HBsAg) and the corresponding fragment of the viral polymerase in chronic virus carriers lacking detectable HBsAg in serum" JOURNAL OF GENERAL VIROLOGY, Bd. 81, Nr. 5, Mai 2000 (2000-05), Seiten 1165-1174, XP002300766 ISSN: 0022-1317

## Beschreibung

Die Erfindung betrifft Sequenzen einer neuen Mutante oder Variante des Hepatitis B surface Antigens (HBsAg) und Methoden, diese Genom- und Protein-Variante sowie dagegen gerichtete Antikörper aus Patientenproben zu detektieren.

Die neuen Sequenzen führen zu 5 noch nicht bekannten Aminosäure-Austauschen in dem Hepatitis B surface Antigen, HBsAg in den Aminosäurepositionen 115 bis 181 der Aminosäuresequenz des surface Antigens, wobei sich 4 Substitutionen in der Region der a-Determinante befinden (aa 101 bis aa 180) sowie 1 Substitution in unmittelbarer Nachbarschaft davon (aa 181).

Die Erfindung betrifft auch immunchemische Nachweisverfahren zum gleichzeitigen Nachweis dieser neuen HBV - Variante zusammen mit bekannten Varianten/Subtypen sowie die Verwendung der neuen Sequenzen in Verbindung mit bekannten Sequenzen zum gleichzeitigen Nachweis von HBV-spezifischen Antikörpern. Die Antigen bzw. Antikörper-Bestimmungen können jeweils in einem Testansatz differenzierend oder nicht-differenzierend durchgeführt werden.

Schließlich betrifft die Erfindung auch den Nachweis der entsprechenden Nukleinsäuren mit Hilfe sogenannter Nukleinsäure-Tests (z.B. Polymerase Chain Reaction, PCR) mit Hilfe geeigneter Primer sowie die Verwendung der neuen Aminosäuresequenzen zur Erzeugung von Impfstoffen.

Das Hepatitis B Virus ist bekanntermaßen der Auslöser einer Vielzahl von Erkrankungs-Verläufen von milden inapparenten Infektionen bis hin zu chronisch aktiven und fulminant verlaufenden durch virale Infektionen ausgelösten Leberentzündungen (Virushepatitiden). Die chronische Infektion mit HBV stellt mit geschätzten 400 Millionen betroffenen Menschen ein globales Gesundheitsproblem dar (Lee, N. Engl. J. Med. 337; 1733-1745 (1997)).

Als geeignetste Prophylaxe für die weltweit häufig anzutreffende HBV - Infektion gilt die aktive Immunisierung (Stimulation der Antikörperantwort durch Antigengabe) und auch die passive Immunisierung (durch Injektion präformierter Antikörper).

Das HBV gehört zu den Hepadna-Viren und stellt ein Viruspartikel mit einem Durchmesser von 42 nm dar, das aus Kern und Hülle besteht. Das Genom des Virus ist eine doppelsträngige, ringförmige DNA- Sequenz von etwa 3200 Nukleotiden, die mindestens sechs verschiedene virale Gene kodieren (Tiollais et al., Nature 317: 489-495 (1985)).

Es liegen vier offene Leserahmen für die Bildung der viralen Proteine vor.

Im S-Gen liegt die Information für das HBV surface Antigen (HBsAg), das auch small protein (S) genannt wird. Daneben gibt es größere Formen, die als large protein (L) und middle protein (M) bezeichnet werden. Allen drei Proteinen gemeinsam ist die 226 Aminosäuren umfassende S-HBsAg Sequenz (Gerlich et al., Viral Hepatitis and Liver Disease, Hollinger et al., William-Wilkens, Baltimore, MD, pages 121-134 (1991)). Die Proteinregionen vor dem small HBs werden auch Pre-S1 und Pre-S2 bezeichnet, umfassen 108 bzw. 55 Aminosäuren und sind beide in dem L-Protein (389 Aminosäuren) enthalten, während das M-Protein nur das Pre-S2 zusammen mit dem S-Antigen umfasst (281 Aminosäuren). Die Pre-S Proteine weisen unterschiedliche Glykosilierungsgrade auf und tragen die Rezeptoren für die Erkennung der Leberzellen. Sofern nicht anders angegeben, beziehen sich die Aminosäurepositionen in dieser Anmeldung auf das S-Antigen (226 aa) ohne Pre-S1- und ohne Pre-S2-Region.

Das C-Gen trägt die Information für das Nukleokapsid Protein, Hepatitis B Core Antigen (HBcAg). Die Translation dieses Proteins kann bereits in der Pre-C-region starten und zur

Bildung von Hepatitis Be-Antigen (HBeAg) führen. Das HBeAg weist gegenüber HBcAg eine andere Faltung und Immunogenität auf. HBeAg kommt im Gegensatz zu HBcAg frei im Serum vor und wird bei positivem Nachweis als Indikator für die Bildung von HBcAg und damit für die Bildung infektiöser Viruspartikel angesehen.

Die im Viruspartikel enthaltene Reverse Transkriptions DNA-Polymerase wird von P-Gen codiert und für das Transaktivator X-Gen wird eine ursächliche Rolle bei der Entstehung von HBV-assoziierten primären Leberzell-Karzinomen diskutiert.

Der virale Replikationszyklus von HBV umfasst eine intrazelluläre pre-genomische RNA, die im viralen Nukleocapsid in die DNA umgeschrieben wird. Da die HBV - eigene Reverse Transkriptase DNA- Polymerase über keine Richtigkeits-Lesefähigkeit verfügt (proof-reading capability), werden mit relativ hoher Häufigkeit falsche Nukleotide eingebaut. Als Folge weist das HBV eine Mutationsrate auf, die mit ca. 1 Nukleotid/10000 Basen/Infektonsjahr etwa dem 10-fachen dessen entspricht, was andere DNA Viren aufweisen (Blum, Digestion 56: 85-95 (1995); Okamoto et al., Jpn. J. Exp. Med. 57: 231-236 (1987)).

Daneben treten auch recht häufig Deletionen und Insertionen auf (Carman et al., Lancet 341: 349-353 (1993)).

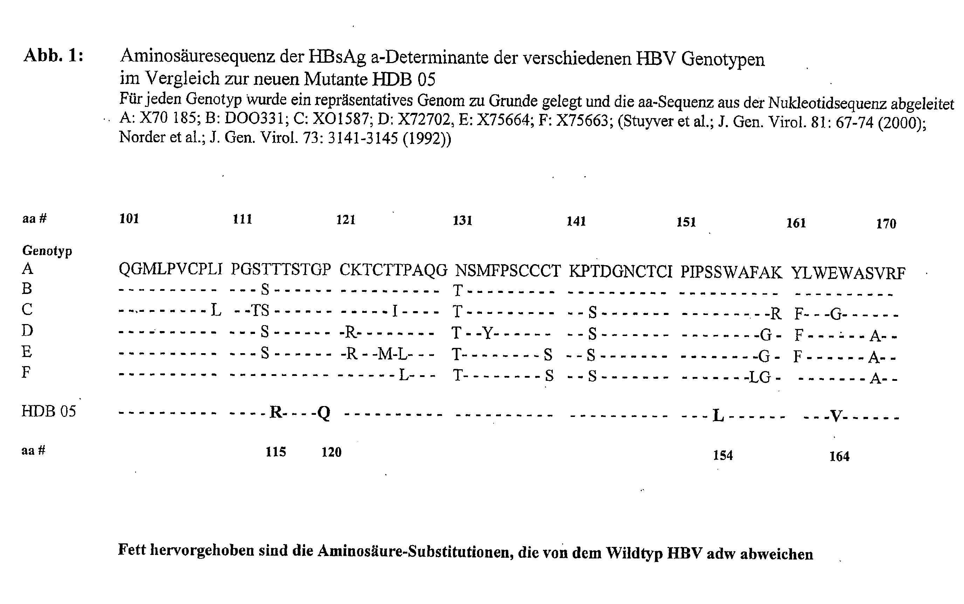

Die resultierende Variabilität von HBV drückt sich unter anderem in dem Auftreten von 9 serologisch definierten Subtypen (Courouce et al., Bibliotheca Haematologica 42: 1 (1976) und insgesamt mindestens 6 verschiedenen Genotypen aus, die mit A bis F bezeichnet werden (Abb. 1) und eine geographische Verteilung aufweisen. (Norder et al., J. Gen. Virol. 73: 3141-3145 (1992), Norder et al., Virology 198: 489-503 (1994)).

Außerdem werden eine Reihe von Mutanten beschrieben, bei denen 1 Aminosäure oder mehrere ausgetauscht vorliegen, fehlen oder überzählig sind.

Neben natürlicherweise stattfindenden Mutationen (Cooreman et al., Hepatology 30: 1287-1292 (1999) kann eine Gabe von HBV Immunglobulinen und/oder eine antivirale Therapie (z.B. mit Lamivudine) einen sogenannten Selektionsdruck ausüben, was zum vermehrten Auftreten sogenannter "Escape-Mutanten" führen und die Auftretens-Wahrscheinlichkeit von HBV-Mutanten deutlich erhöhen kann (Terrault et al., Hepatology 28: 555-561 (1998); Tillmann et al., Hepatology 30: 244-256 (1999); Hunt et al., Hepatology 31: 1037-1044 (2000).

Nicht alle HBV-Mutationen führen zu replikationsfähigen Viren und oft liegt eine Coexistenz mit replikationsfähigen Virus vor, was auch die Sequenzierungs-Genauigkeit von isolierter DNA limitiert oder gar zur Nichterkennung von veränderten Sequenzen durch PCR, Klonierungsarbeiten mit anschließender Sequenzierung führt, wenn diese quantitativ < 10 % der Gesamt-DNA ausmachen (Cooreman et al., J. Biomed. Sci. 8: 237-247 (2001).

Demnach ist die Isolierung von Mutanten vorteilhaft, wobei die sich anschließende Identifizierung und Charakterisierung einzelner Mutanten möglicherweise zu verbesserten Vakzinen und Diagnostika führt.

Die Immunantwort nach einer Infektion mit HBV ist hauptsächlich gegen die sogenannte a-Determinante als eine allen Hepatitis B Viren gemeinsame Region des S-Proteins gerichtet, die sich auf der Oberfläche der Viruspartikel befindet (Gerlich et al., supra) und die den heterogensten Teil der B-Zell-Epitope des S-Gens darstellen.

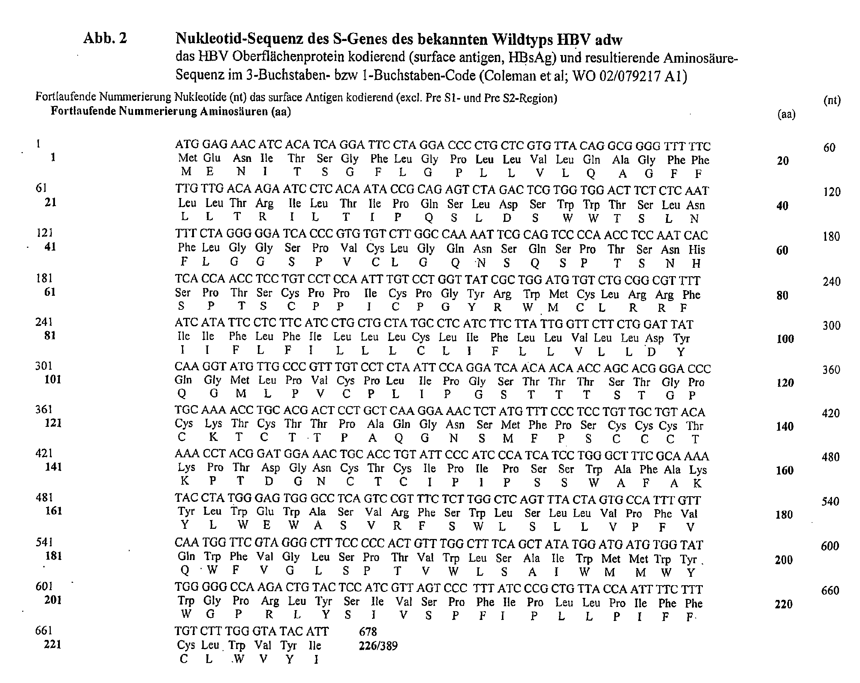

Als Bindestellen für Antikörper werden nach heutigem Kenntnisstand insgesamt mindestens 5 sich teilweise überlappende Epitope auf der a-Determinante zwischen Aminosäure-Position 101 und 180 angenommen (Abb. 1 und 2) wie durch die Anwendung von monoklonalen Antikörpern gezeigt werden konnte (Peterson et al., J. Immunol. 132: 920-927 (1984)).

Es handelt sich hauptsächlich um komplexe Konformations-Epitope, die durch mehrere Disulfid-Brücken stabilisiert werden. Teilweise liegen auch Sequenz-Epitope vor, die mit Hilfe synthetisch hergestellter zyklischer Peptid-Strukturen dargestellt werden können.

Sogenannte "schützende Antikörper", die nach einer natürlichen Infektion mit HBV im Serum zirkulieren, sind zu 99 % gegen die sehr immunogene a-Determinante des HBV gerichtet (Jilg, Vaccine 16: 65-68 (1998).

Auf diese Tatsache stützt sich die breite Anwendung der Immunisierung mit Vakzinen, die entweder aus Humanserum isoliert oder gentechnologisch hergestellt wurden und die Verabreichung von Hepatitis B Immunglobulinen, die humane HBV - spezifische Antikörper enthalten. Beide prophylaktischen Strategien beruhen auf dem neutralisierenden Effekt, die HBs-spezifische Antikörper nach Bindung an die "a-Loop-Epitope" entfalten (Carman et al., Hepatology 24: 489-493 (1996), Muller et al., J. Hepatol. 13: 90-96 (1991) und Samuel et al., N. Engl. J. Med. 329: 1842-1847 (1993)).

Ähnlich beruhen heute weit verbreitete Diagnostika auf der Bindung von a-Determinantenspezifischen Antikörpern mit Epitopen der a-Determinante.

So wird bei der im Blutspendewesen weltweit angewendeten HBsAg-Bestimmung mit immunchemischen Bestimmungsmethoden im Serum von Spendern zirkulierendes HBV Oberflächenantigen mit Antikörpern gegen die a-Determinante (ployklonal oder monoklonalen Ursprungs) nachgewiesen und bei positivem Resultat die entsprechende Blutspende verworfen, um iatrogene HBV Infektionen durch HBV-kontaminiertes Blut zu verhindern. Eine weitere Anwendung der HBsAg-Bestimmung liegt im Nachweis einer vorliegenden akuten HBV-Infektion. Umgekehrt wird mit der Bestimmung von HBs-spezifischen Antikörpern im Blut von Probanden mit einem positiven Bestimmungsresultat von HBsAg-spezifischen Antikörpern (Anti-HBs) nachgewiesen, dass entweder eine natürliche Infektion abgelaufen oder dass eine durchgeführte Vakzinierung erfolgreich verlaufen ist.

Schließlich beruht auch die Nukleinsäure-Testung z.B. mit Hilfe der Polymerase-Chain-Rektion (PCR, Polymerase-Ketten-Reaktion) auf der Verwendung von Primern (Starter), die für die HBV Nukleotide spezifisch sind.

Auf Grund der zentralen Rolle, die die a-Determinante bei der aktiven Immunisierung (Vakzinierung mit HBV Antigen), der passiven Immunisierung (Schutz durch HBV -spezische Immunglobuline), dem Nachweis von Impferfolg bzw. stattgefundener HBV Infektion (beides mittels Bestimmung von HBsAgspezischen Antikörpern, Anti-HBs)und schließlich der Sicherheit im Blutspendewesen (HBsAg-Bestimmung und PCR), ist verständlich, dass in Fachkreisen das Auftreten von Mutanten und auch neuen Varianten mit großer Aufmerksamkeit verfolgt wird.

Als Konsequenz könnten in der a-Determinante des HBV veränderte aber replikationsfähige neue Mutanten und/oder Varianten sowohl das prophylaktische als auch das diagnostische Konzept unterlaufen (Brind et al., J. Hepatol. 26: 228-235 (1997), Fischer et al., Transplant Proc. 31: 492-493 (1999), Ghany et al., Hepatology 27: 213-222 (1998), Protzer-Knolle et al., Hepatology 27: 254-263 (1998), Carman et al., Gastroenterology 102: 711-719 (1992) und Coleman et al., WO 02/079217 A1, (2002)). Die EP- 1 142 906 A1 beschreibt eine HBsAg-Variante, die an Position 120 Glutamin anstelle von Prolin aufweist. Cooreman MP et al., Hepatology 30: 1287-1292 (1999) offenbaren Antikörper, die die an HBsAg-Mutanten und ebenso an Wildtyp-HBsAg binden. Darüber hinaus wurde eine Publikation zum murinen Urokinase-Typ Plasminogen Aktivator-Gen gefunden, worin ein Polypeptid offenbart wird, das zufällig einem kurzen Abschnitt der vorliegend offenbarten mutierten HBsAg-Sequenz entspricht (Degen SJ et al., Biochemistry 26: 8270-8279 (1987)).

Die Abgrenzung von Varianten und Mutanten des HBV ist nicht scharf, wobei ein diesbezüglicher Vorschlag breite Anwendung findet (Carman, J. Viral Hepat. 4 (suppl.1): 11-20 (1997).

Dem zu Folge sollte die Bezeichnung "Variante" für natürlicherweise vorkommende Subtypen angewendet werden, die ohne bekannte Interferenz durch Selektionsdruck (antivirale Therapie und / oder Immunglobulin-Gabe) auftreten und ein geographisches Verteilungsmuster aufweisen.

Die Charakterisierung und anschließende Klassifizierung der Subtypen erfolgt mit Hilfe monoklonaler Antikörper und basiert auf einem veränderten Reaktionsmuster auf Grund des Austausches von einer oder wenigen Aminisäure(n). Die Grundlage der Klassifizierung stellen die Aminosäurepositionen 122 oder 160 der verbreitetsten HBV Sequenz dar: aa 122 und aa 160 =Lysin, K.

Alle Serotypen enthalten die Gruppen-spezifische a-Determinante, während die aa 122 und zusätzlich 133 und 134 den d- bzw. r-Subtyp und aa 160 die Zugehörigkeit zum w- bzw r-Subtyp bestimmen. Auf dieser Basis lassen sich HBV Subtypen grob in adr, adw, ayr oder ayw einteilen, die sich weiter in mindestens 9 Sub-Subtypen unterscheiden lassen: ayw1, ayw2, ayw3, ayw4, ayr, adwr2, adw4, adrq+ und adrq- (Swenson et al., J. Virol. Meth. 33: 27-28 (1991), Blitz et al. J. Clin. Microbiol. 36: 648-651, Ashton-Rickardt et al., J. Med. Virol. 29: 204-214 (1989)).

Da diese Klassifizierung eine serologische Reaktivität zur Grundlage macht, muß nicht jede Typisierung notwendigerweise eine Variabilität auf der Aminosäure-Ebene bedeuten, weshalb dem Genotyping auf der S-Gen-Ebene der Vorzug gegeben wird (Ohba et al., Virus Res. 39: 25-34 (1995).

Subtypen treten aus noch nicht bekannten Gründen in bestimmten geographischen und ethnischen Mustern auf.

Die Bezeichnung Mutation sollte nach Carman Varianten vorbehalten bleiben, die ausschließlich unter Selektionsdruck wie Vakzinierung oder antivirale Therapie entstehen. Es sind bereits viele Mutationen beschrieben worden, von denen manche zu diagnostisch falschen Befundungen führten (Carman et al., Lancet 345: 1406-1407) und von denen die nachstehend genannten aa-Austausche beispielhaft zitiert werden:

| Consensus: | aa-Position | Mutante: |
|---|---|---|
| l | 110 | V |
| P | 111 | T |
| T | 114 | S |
| T | 116 | S |
| P | 120 | T/S |
| T | 123 | A/N |
| I/T | 126 | A/S |
| Q | 129 | H/R |
| K/M | 133 | L |
| T | 143 | M/L |
| D | 144 | H/A/E |
| G | 145 | R/A |
| A | 157 | R |

sowie Cystein-Austausche in den aa-Positionen 107, 124, 137, 147 & 149. (Coleman, supra; Okamoto et al., Pediatr. Res. 32: 264-268 (1992); Zhang et al., Scand. J. Infect. Dis. 28: 9-15 (1996); Zuckermann et al., Lancet 343: 737-738 (1994)).

Überraschend wurde in einer Humanprobe eines an Leberentzündung erkrankten Patienten aus Frankreich (interne Nummer: 119617) ein atypisches Reaktionsmuster von Hepatitis-Markern gefunden.

Neben dem Krankheitsbild mit Erhöhung der für eine derartige Infektion typischen Leberwerte weisen auch nachgewiesene Hepatitis Core Antikörper der IgM-Klasse auf eine akute HBV-Infektion hin, ohne dass allerdings der HBsAg-Nachweis mit einem zugelassenen leistungsstarken HBsAg-ELISA gelang.

Eine durchgeführte PCR ergab überraschend bei der Probe ein positives Resultat und das Sequenzierungsergebnis führte völlig überraschend zu der in Abb. 3 und 4 dargestellten Nukleotid-Sequenz und der in Abb. 5 und 6 abgebildeten Aminosäre-Sequenz, die beide unerwartet zu dem beschriebenen Substitutionsmuster führten.

Aus diesen Sequenzen wird deutlich, dass es sich völlig überraschend nicht um eine Punktmutation, d.h. Austausch weniger Nukleotide und auch nicht um einen möglicherweise serologisch zu charakterisierenden Subtyp handelt, da insgesamt n=5 Aminosäuren in der Region von aa 115 bis 181 gegenüber dem Genotyp A substituiert vorliegen. Im Hinblick auf die Häufigkeit der Aminosäure-Substitutionen ist überraschend davon auszugehen, dass es sich um eine neue Mutante handelt oder dass die Mutationen so ausgeprägt sind, dass die Konsequenz eher als neue Variante zu beschreiben ist, die im Folgenden als HDB 05-Variante bezeichnet wird.

Die Analyse der besten Überstimmung der Aminosäuresequenz der a-Determinante mit bekannten Sequenzen verweist auf Genotyp A (Abb. 1), Subtyp adw (Abb. 2) von dem sich die neue Variante überraschend allerdings in 4 aa-Positionen unterscheidet. Prominentestes Merkmal sind die 2 benachbarten Substitutionen in der Region zwischen aa 115 und 120 und zwischen aa 154 und 164 sowie die aa-Position # 181 in unmittelbarer Nachbarschaft der a-Determinante gemäß Abb. 1, 5 und 6.

Da bekannt ist, dass Epitope auf der a-Determinante strukturbedingt sind, das heißt als sogenannte Konformationsepitope vorliegen können, ist es naheliegend, dass die Immunogenität und auch die Bindefähigkeit von Antikörpern an die a-Determinante durch den Aminosäure-Austausch in Position # 181 beeinflußt werden kann.

Schließlich wurde völlig überraschend eine Identität der Nukleotid- und Aminosäure-Sequenz von Serumprobe # 119617 mit den entsprechenden Analysenresultaten einer unabhängigen anderen Serumprobe aus Österreich festgestellt (interne Nummer: 118457), die ebenfalls von einem an Leberentzündung erkrankten Patienten stammt. Daraus kann geschlossen werden, dass es sich bei HDB 05 um eine replikationsfähige und infektiöse Mutante bzw. Variante von HBV handelt, die eine gewisse Verbreitung gefunden haben kann.

Die vorliegende Erfindung betrifft daher ein Oligo- oder Polypeptid umfassend eine Aminosäuresequenz, die eine Teilsequenz von SEQ ID NO:12 mit wenigstens 70 aufeinanderfolgenden Aminosäuren von SEQ ID NO:12 ist, wobei die Teilsequenz die Positionen 73, 78, 112, 122 und 139 von SEQ ID NO:12 einschließt. Diese Aminosäurepositionen entsprechen den Positionen 115, 120, 154, 164 und 181 des S-Antigens von HBV. In weiteren Ausführungsformen umfasst die Teilsequenz wenigstens 75, wenigstens 80, wenigstens 85, wenigstens 90, wenigstens 95 oder wenigstens 100 aufeinanderfolgende Aminosäuren der in SEQ ID NO:12 gezeigten Aminosäuresequenz.

Das erfindungsgemäße Polypeptid kann auch ein Fragment eines HBs-Antigens eines Hepatitis B-Virus umfassen, wobei das HBs-Antigen an Position 115 Arginin, an Position 120 Glutamin, an Position 154 Leucin, an Position 164 Valin und an Position 181 Arginin aufweist, und das Fragment Arginin 115, Glutamin 120, Leucin 154, Valin 164 und Arginin 181 umfasst.

Die Gesamtlänge des Oligo- oder Polypeptids entspricht in der Regel bis zu 1000 Aminosäuren, vorzugsweise bis 500 Aminosäuren, bevorzugter bis 300 Aminosäuren, am bevorzugtesten bis 200 Aminosäuren. Die Oligo- oder Polypeptide können auch Fremdaminosäuren enthalten, die nicht vom Genom eines Hepatitis B-Virus kodiert werden. So können Aminosäuren enthalten sein, die die Kopplung an feste Phasen erleichtern oder die Kopplung an Markierungssubstanzen ermöglichen. Es können Aminosäuren enthalten sein, die aufgrund der Klonierung entstanden sind und bei der rekombinanten Expression mit exprimiert wurden. Schließlich kann das erfindungsgemäße Oligo- oder Polypeptid ein Fusionsprotein sein, das neben von HBV abgeleiteten Aminosäuren einen Fusionspartner enthält, z. B. eine "tag"-Sequenz, die die Reinigung erleichtert, oder ein Proteinanteil, der die Löslichkeit und/oder Ausbeute bei der rekombinanten Expression erhöht. Derartige Fusionspartner sind dem Fachmann an sich bekannt.

In einer anderen Ausführungsform enthalten die Oligo- oder Polypeptide keine Fremdaminosäuren, die nicht vom Genom eines HBV kodiert werden. Entsprechend bestehen diese Oligo- oder Polypeptide aus einer der oben und/oder in den Ansprüchen beschriebenen Aminosäuresequenzen.

Das erfindungsgemäße Oligo- oder Polypeptid ist vorzugsweise immunogen, d.h. es kann eine Antikörperantwort in einem Säugerorganismus induzieren. Üblicherweise enthält das Oligo- oder Polypeptid wenigstens eine antigene Determinante oder wenigstens ein Epitop. In einer besonderen Ausführungsform enthält das Oligo- oder Polypeptid ein Epitop, das in anderen HBV-Varianten, z. B. in Genotyp A; Subtyp adw, nicht enthalten ist.

Vorzugsweise umfasst das Oligo- oder Polypeptid eine der Aminosäuresequenzen SEQ ID NO:12 und SEQ ID NO:13.

Ein weiterer Aspekt der Erfindung ist ein immunogenes Peptid oder eine Mischung immunogener Peptide, enthaltend eines oder mehrere der in dieser Anmeldung beschriebenen Oligo- oder Polypeptide. Das immunogene Peptid oder die immunogene Mischung kann das/die Oligo- oder Polypeptide alleine oder in Verbindung mit bekannten HBV-Immunogenen enthalten.

Gegenstand der vorliegenden Erfindung sind auch Nukleinsäuremoleküle, die vom Genom der neuen HBV-Variante HDB 05 abgeleitet sind, insbesondere Nukleinsäuremoleküle, die von dem Gen abgeleitet sind, das HBsAg kodiert.

Die Erfindung betrifft daher weiterhin ein Oligo- oder Polynukleotid umfassend eine Nukleotidsequenz, die eine Teilsequenz von SEQ ID NO:1 mit wenigstens 200 aufeinanderfolgenden Nukleotiden von SEQ ID NO:1 ist, wobei die Teilsequenz die Positionen 218, 233, 335, 365 und 416 von SEQ ID NO:1 einschließt. In weiteren Ausführungsformen umfasst die Teilsequenz wenigstens 250 oder wenigstens 300 aufeinanderfolgende Nukleotide der in SEQ ID NO:1 gezeigten Nukleotidsequenz.

In einer anderen Ausführungsform umfasst das Oligo- oder Polynukleotid eine Nukleotidsequenz, die unter stringenten Bedingungen, vorzugsweise spezifisch, mit einem zu der Sequenz SEQ ID NO:1 komplementären Polynukleotid hybridisiert. Verfahren zur Bestimmung, ob eine gegebenes Oligo- oder Polynukleotid mit einem anderen Polynukleotid hybridisiert, sind dem Fachmann an sich bekannt. Ein spezielles Beispiel für "stringente Bedingungen" sind folgende Bedingungen: a) 16-stündige Inkubation bei 42 °C in einer Lösung enthaltend 50 % Formamid, 5xSSC (150 mM NaCl, 15 mM Trinatriumcitrat), 50 mM Natriumphosphat pH 7,6, 5xDenhardt's Lösung, 10% Dextransulfat und 20 µg/ml denaturierte, gescherte Lachssperma-DNA; b) anschließend Waschen in 0,1xSSC bei ungefähr 65°C. Hybridisierungs- und Waschbedingungen sind dem Fachmann an sich bekannt und beispielhaft in Sambrook et al., Molecular Cloning: A Laboratory Manual, Second Edition, Cold Spring Harbor, N.Y., (1989) angegeben. Eine Nukleotidsequenz hybridisiert spezifisch an ein gegebenes Polynukleotid, wenn sie nicht oder wesentlich schwächer an andere Nukleotidsequenzen hybridisiert. Im vorliegenden Fall kann das bedeuten, dass die Nukleotidsequenz nicht oder nur schwach an für HBsAg kodierende Polynukleotide aus herkömmlichen HBV-Varianten (z. B. Genotyp A, Subtyp adw) hybridisiert.

Die Erfindung betrifft auch ein Oligo- oder Polynukleotid umfassend eine Nukleotidsequenz, die für ein erfindungsgemäßes Oligo- oder Polypeptid kodiert, wie es in dieser Anmeldung beschrieben ist. Ein weiterer Aspekt der Erfindung ist ein Oligo- oder Polynukleotid umfassend eine zu den oben beschriebenen Nukleotidsequenzen komplementäre Nukleotidsequenz.

Die Gesamtlänge des Oligo- oder Polynukleotids entspricht in der Regel bis zu 3000 Nukleotiden, vorzugsweise bis 1500 Nukleotiden, bevorzugter bis 900 Nukleotiden, am bevorzugtesten bis 600 Nukleotiden. Die Oligo-oder Polynukleotide können auch Nukleotide enthalten, die nicht vom Genom eines Hepatitis B-Virus herstammen. So können Nukleotide enthalten sein, die für bestimmte Aminosäuren kodieren, die gewünschte Funktionen erfüllen sollen, wie oben beschrieben. Es können Nukleotide enthalten sein, die aufgrund der Klonierung entstanden sind, z. B. um bestimmte Schnittstellen einzuführen. Schließlich kann das erfindungsgemäße Oligo- oder Polynukleotid für ein Fusionsprotein kodieren, das neben von HBV abgeleiteten Aminosäuren einen Fusionspartner enthält, z. B. eine "tag"-Sequenz, die die Reinigung erleichtert, oder ein Proteinanteil, der die Löslichkeit und/oder Ausbeute bei der rekombinanten Expression erhöht. Derartige Fusionspartner und die sie kodierende DNA sind dem Fachmann an sich bekannt.

Bevorzugte Oligo- oder Polynukleotide der vorliegenden Erfindung umfassen eine Nukleotidsequenz, die ausgewählt ist aus der Gruppe bestehend aus SEQ ID NO:1 und SEQ ID NO:2.

Die erfindungsgemäßen Polynukleotide können auch markiert sein, beispielsweise durch eine Fluoreszenzmarkierung oder eine radioaktive Markierung. Derartige Polynukleotide können vorteilhaft in einer Hybridisierungsreaktion oder einer Polymerasekettenreaktion (PCR) eingesetzt werden.

Die Erfindung betrifft auch einen Vektor oder ein Plasmid enthaltend ein Oligo- oder Polynukleotid gemäß der Erfindung. Das Plasmid kann beispielsweise ein Klonierungsvektor sein, der dazu dient, die Nukleinsäure in Wirtszellen zu vermehren oder bestimmte Restriktionsschnittstellen zur Verfügung zu stellen. Expressionsvektoren sind Vektoren, die die Expression der klonierten Nukleinsäure in Wirtszellen erlauben. Wirtszellen können verschiedene prokaryontische oder eukaryontische Zellen sein. Prokaryontische Wirtszellen sind z. B. bakterielle Zellen wie *E. coli*-Zellen. Die erfindungsgemäßen Expressionsvektoren können bestimmte Steuerelemente wie z.B. Promotoren oder Bindungsstellen für Repressionsfaktoren enthalten. In einer weiteren Ausführungsform enthalten die Expressionsvektoren einen Nukleinsäureabschnitt, der für einen Teil eines Fusionsproteins kodiert.

Die Erfindung betrifft ebenfalls eine Zelle, z. B. eine Wirtszelle, die ein erfindungsgemäßes Polynukleotid, Plasmid oder einen erfindungsgemäßen Vektor enthält. Die Wirtszellen können unter geeigneten Bedingungen kultiviert werden, so dass eine Transkription der enthaltenen Nukleinsäure und nachfolgende Translation stattfindet. Die Erfindung betrifft auch ein Verfahren zur Herstellung eines Polypeptids, bei dem man ein Polynukleotid, ein Plasmid oder einen Expressionsvektor der Erfindung in Wirtszellen einbringt und die Wirtszellen unter Bedingungen kultiviert, die zur Expression des Polypeptids führen. Gegebenenfalls kann anschließend das Polypeptid aus den Wirtszellen gewonnen werden. Vorzugsweise findet die Herstellung des Polypeptids in Bakterien, am bevorzugtesten in *E. coli*-Zellen statt. Geeignete Mittel und Bedingungen zur Kultivierung sind beispielsweise in Ausubel et al. (1993) "Current Protocols in Molecular Biology" beschrieben. Die Gewinnung des exprimierten Polypeptids geschieht nach für den Fachmann an sich bekannten Methoden. Verschiedene Verfahren zur Proteinreinigung sind beispielsweise beschrieben in Scopes R. (1994) "Protein Purification: Principles and Practice" (3rd edition) Springer Verlag.

Die Polypeptide und Peptide der vorliegenden Erfindung können jedoch auch chemisch durch bekannte Verfahren wie z.B. Festphasensynthese hergestellt werden. Ebenso können die erfindungsgemäßen Polynukleotide durch bekannte Verfahren der chemischen Synthese hergestellt werden. Durch chemische Synthese erhaltene Polynukleotidfragmente können dann auch enzymatisch durch Ligasen verknüpft werden. Die erfindungsgemäßen Oligo-oder Polynukleotide können auch durch "site-directed mutagenesis" aus bekannten Sequenzen hergestellt werden, indem an bestimmten Positionen Punktmutationen eingeführt werden. Derartige Verfahren sind dem Fachmann an sich bekannt.

Ein weiterer Gegenstand der Erfindung ist ein anti-idiotypischer Antikörper, der eine Aminosäuresequenz eines erfindungsgemäßen Oligo- oder Polypeptids repräsentiert. Verfahren zur Herstellung antiidiotypischer Antikörper sind dem Fachmann an sich bekannt.

Die Erfindung betrifft auch einen Testkit zum Nachweis von Hepatitis B-Viren, enthaltend ein erfindungsgemßes Oligo- oder Polypeptid, ein erfindungsgemäßes Oligo- oder Polynukleotid.

Die Erfindung bezieht sich auch auf ein immunogenes Peptid oder eine Mischung immunogener Peptide, enthaltend ein oder mehrere erfindungsgemäßen Oligo- oder Polypeptide alleine oder in Verbindung mit bekannten HBV-Immunogenen.

Eine spezielle Ausführung eines Immunoassays stellt der sogenannte Enzymimmunoassay dar, von dem ein mögliches Testprinzip im folgenden beispielhaft beschrieben wird, ohne jedoch die erfindungsgemäße Idee darauf zu beschränken:

Bei dem sehr weit verbreiteten sogenannten Sandwich-Prinzip werden auf einem geeigneten Träger (z.B. Mikropartikel oder Oberfläche von Vertiefungen einer Mikrotitrationsplatte) immobilisierte Antikörper oder Fragmente davon mit der Untersuchungsprobe inkubiert. An die Antikörper gebundenes HBsAg wird nach Entfernen überschüssiger Probe detektiert, indem eine weitere Inkubation erfolgt mit Anti-HBs-Antikörpern (monoklonal oder polyklonal oder Fragmente bzw. Mischungen dieser Fragmente), die mit einer Sonde versehen sind. Als Sonde wird häufig ein Enzym eingesetzt, dessen katalytische Umsetzung (nach Entfernen des überschüssigen Reagenzes) eines geeigneten Substrates zu einer Farbreaktion führt, die photometrisch gemessen wird und deren Intensität dem in der Probe vorhandenen HBsAg-Gehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. Das Biotin/Streptavidin-System).

All diese Ausführungsformen entsprechen dem Stand der Technik, so dass unter "Bestimmung von HBsAg der neuen HBV Variante" vorliegend alle Verfahren verstanden werden, die sich zum Nachweis von Polypeptidsequenzen oder Antigenen der neuen HBV Variante eignen, ungeachtet ob alleine das HBsAg der neuen Variante bestimmt wird oder in Verbindung mit HBsAg von bekannten a-Determinanten und/oder bekannten Mutationen in der a-Region.

Ebenso ist es möglich, aus ökonomischen Gründen eine HBsAg-Bestimmung mit einer Nachweismethode gegen einen weiteren Analyten (z.B. HIV-Antigen oder die gleichzeitige Bestimmung von HBV Varianten-HBsAg und dagegen gerichtete spezifische Antikörper) in einem Testansatz (differenzierend oder nicht-differenzierend) zu kombinieren.

Die Erfindung betrifft auch ein Verfahren zum Nachweis von Antikörpern, die gegen ein Hepatitis B-Antigen gerichtet sind, dadurch gekennzeichnet, dass (a) eine Probe mit einem erfindungsgemäßen Oligo- oder Polypeptid unter Bedingungen inkubiert wird, die die Bildung eines Antigen-Antikörper-Komplexes gestatten; und (b) der Antikörper-Antigen-Komplex, der das Oligo- oder Polypeptid enthält, nachgewiesen wird.

Eine spezielle Ausführung stellt der sogenannte Enzymimmunoassay dar, von dem ein mögliches Testprinzip im folgenden beispielhaft beschrieben wird, ohne jedoch die erfindungsgemäße Idee darauf einzuschränken:

Bei dem sehr weit verbreiteten sogenannten Sandwich-Prinzip werden auf einem geeigneten Träger (z.B. Mikropartikel oder Oberfläche von Vertiefungen einer Mikrotitrationsplatte) immobilisierte Epitop-tragende Polypeptid- oder Protein-Sequenzen mit der Untersuchungsprobe inkubiert. An die Epitope gebundene Antikörper werden nach Entfernen überschüssiger Probe detektiert, indem eine weitere Inkubation erfolgt mit Epitop-tragenden Polypeptid- oder Protein-Sequenzen, die mit einer Sonde versehen sind. Als Sonde wird häufig ein Enzym eingesetzt, dessen katalytische Umsetzung (nach Entfernen des überschüssigen Reagenzes) eines geeigneten Substrates zu einer Farbreaktion führt, die photometrisch gemessen wird und deren Intensität dem in der Probe vorhandenen Antikörpergehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. Das Biotin/Streptavidin-System).

Ebenso können die Polypeptidstrukturen der HBV Variante durch anti-idiotypische Antikörper dargestellt werden oder durch Wahl eines geeigneten Testprinzips auch Variantenspezifische monoklonale oder polyklonale Antikörper zur Bestimmung von Anti-HBs-Antikörpern (z.B. in einem kompetitiven Testformat) herangezogen werden. Es ist ebenso bekannt, dass durch Wahl des Testprinzips auch eine Differenzierung der Immunglobulin-Klassen erfolgen kann (z.B. durch das "indirekte" Verfahren mit einem zweiten Klassenspezifischen Antikörper (z.B. IgG oder IgM spezifisch) mit Sonde oder mit Hilfe des sogenannten Anti-µ-Prinzips (IgM spezifisch). Natürlich müssen die Methoden und Materialen (inkl. Sonde und Polypeptidsequenzen) dem jeweiligen Ziel angepaßt werden.

All diese Ausführungsformen entsprechen dem Stand der Technik, so dass unter "Bestimmung von Antikörpern, die für die a-Determinante der neuen HDB 05-Variante spezifisch sind" mit der vorliegenden Erfindung alle Verfahren verstanden werden, die sich zum Nachweis von Immunglobulinen und/oder Immunglobulin-Klassen gegen die neue HBV Variante eignen, ungeachtet ob alleine der Antikörper gegen die neue Variante gesucht wird oder in Verbindung mit Antikörpern gegen bekannte a-Determinanten und / oder bekannte Mutationen in der a-Region.

In einem anderen Verfahren kann eine Hepatitis B-Nukleinsäure nachgewiesen werden. Dieses Verfahren ist dadurch gekennzeichnet, dass (a) eine Probe mit einem erfindungsgemäßen Oligo- oder Polynukleotid unter Bedingungen inkubiert wird, die die selektive Hybridisierung des Oligo- oder Polynukleotids mit einer Hepatitis B-Nukleinsäure in der Probe gestatten; und (b) bestimmt wird, ob Polynukleotidduplexe gebildet wurden, die das Oligo- oder Polynukleotid umfassen.

Die Hepatitis B-Nukleinsäure kann auch dadurch nachgewiesen werden, dass (a) eine Probe mit wenigstens einem erfindungsgemäßen Oligo- oder Polynukleotid unter Bedingungen inkubiert wird, die die selektive Hybridisierung des Oligo- oder Polynukleotids mit einer Hepatitis B-Nukleinsäure in der Probe gestatten; (b) eine Polymerasekettenreaktion durchgeführt wird; und (c) bestimmt wird, ob eine Nukleinsäure amplifiziert wurde.

Die Erfindung betrifft auch die Verwendung eines erfindungsgemäßen Oligo- oder Polynukleotids als Primer und/oder als Sonde. Die vorliegenden Nukleotidsequenzen können benutzt werden, um Primer und/oder Gensonden herzustellen, weshalb auch Kits, enthaltend Primer und/oder Sonden zum Nachweis von HBV Varianten-spezifischer Nukleinsäure entweder alleine oder in Verbindung mit bekannten HBV Nukleotidsequenzen in Untersuchungsproben ebenfalls Gegenstand der Erfindung sind.

Auf Basis der vorliegenden Nukleotidsequenzen können Primer entwickelt werden, die in der sog. "Polymerase Chain Reaction" (PCR) Verwendung finden. Die PCR stellt eine Methode dar, um eine gewünschte Nukleotidsequenz einer Nukleinsäure oder eines Nukleinsäuregemisches zu amplifizieren. Dabei werden die Primer jeweils spezifisch durch eine Polymerase mit der gewünschten Nukleinsäure als Leseraster verlängert. Nach Dissoziation von dem Originalstrang werden neue Primer hybridisiert und wiederum durch die Polymerase verlängert. Durch Wiederholung dieser Zyklen wird eine Anreicherung der gesuchten Zielsequenz-Moleküle erreicht.

In Bezug auf Nukleinsäuretests (NAT) ist es möglich, Nukleotid-Sequenzen der vorliegenden Erfindung zu verwenden, um DNA-Oligomere von 6-8 Nukleotiden oder größer herzustellen, die geeignet sind, als Hybridisierungssonden das virale Genom der hier beschriebenen HBV Variante in Personen nachzuweisen, die möglicherweise die Virus-Variante tragen, oder z.B. im Blutspendewesen Blutkonserven auf Vorhandensein des Varianten-Genoms entweder gezielt oder in Kombination mit dem Nachweis von Nukleotidsequenzen bekannter HBV Varianten und/oder HBV-Mutatanten zu screenen.

Ebenso können auf Basis der gefundenen Nukleotidsequenzen der neuen HBV Variante entsprechende Primer entwickelt werden, die für die neue Variante spezifisch sind oder sowohl die neue Variante als auch im Stand der Technik bekannte Varianten detektieren können.

Ein weiterer Gegenstand der vorliegenden Erfindung ist ein isoliertes Hepatitis B-Virus, das ein HBs-Antigen aufweist, das die SEQ ID NO:12 umfasst. Schließlich betrifft die Erfindung auch Kulturen von Gewebezellen, die mit der erfindungsgemäßen HBV-Variante infiziert sind. Auch eine immunogene Zubereitung, die die attenuierte oder inaktivierte erfindungsgemäße Variante von HBV enthält, ist Gegenstand der Erfindung.

Die Erfindung betrifft auch die Verwendung eines erfindungsgemäßen Oligo- oder Polynukleotids oder eines erfindungsgemäßen Oligo- oder Polypeptids zur Herstellung eines Arzneimittels zur Behandlung oder Vorbeugung einer HBV-Infektion. Insbesondere können die erfindungsgemäßen Oligo- oder Polynukleotide bzw. Oligo- oder Polypeptide zur Herstellung eines Impfstoffs gegen HBV verwendet werden.

Ferner schließt die Erfindung auch eine Vakzine ein umfassend ein Polypeptid der vorliegenden Erfindung und ein gebräuchliches Adjuvans (z.B. Freund sches adjuvans, Phosphat-gepufferte Saline o.ä.). Eine derartige Vakzine kann dazu verwendet werden, um die Bildung von Antikörpern in Säugetieren anzuregen. Ähnlich umfasst die Erfindung ein Partikel, das eine Nicht-Varianten spezifische Aminosäure-Sequenz beinhaltet, die eine Partikel-Bildung induziert zusammen mit einem Epitop-enthaltenden Polypeptid, das spezifisch für die erfindungsgemäße HBV Variante ist.

Die Nukleotidsequenzen der Erfindung können auch dazu verwendet werden, sog. Antisense Oligonukleotide darzustellen (gegebenenfalls für therapeutische Zwecke).

Weitere Aspekte der vorliegenden Offenbarung sind die folgenden Gegenstände (1) bis (21):
(1) Isoliertes Oligo- oder Polynukleotid mit einer der Sequenzen ausgewählt aus der Gruppe von Seq id no:1 bis Seq id no:11: **SEQ ID NO:6**
   **331** CCAGGATCAA CAAGAACCAG TACGGGACAA **360** **SEQ ID NO:8**
   **343** AGAACCAGTA CGGGACAA **360** **SEQ ID NO:11**
   **462** TTGTCCTGGG CTTTCGCAAA ATACCTATGG GTGTGG **495**
(2) Oligo- oder Polynukleotid gemäß (1), das zu jeweils mindestens 65% oder 66% oder 67% oder 68% oder 69% oder 70% oder 71% oder 72% oder 73% oder 74% oder 75% oder 76% oder 77% oder 78% oder 79% oder 80% oder 81% oder 82% oder 83% oder 84% oder 85% oder 86% oder 87% oder 88% oder 89% oder 90% oder 91% oder 92% oder 93% oder 94% oder 95% oder 99% oder 97% oder 98% oder 99% mit einer der Sequenzen ausgewählt aus der Gruppe von Seq id no:1 bis Seq id no:11 identisch ist.
(3) Oligo- oder Polynukleotid gemäß (1) oder (2), das mit einem Oligo- oder Polynukleotid, welches eine zu einer der Sequenzen, ausgewählt aus der Gruppe von Seq id no:1 bis Seq id no:11, komplementäre Sequenz hat, unter stringenten Bedingungen hybridisiert.
(4) Isoliertes Oligo- oder Polynukleotid, welches für HBs-Antigen des Hepatitis B-Virus kodiert und ein Oligo- oder Polynukleotid gemäß einem der Gegenstände (1) bis (3) enthält.
(5) Fragment eines Oligo- oder Polynukleotids, welches für HBs-Antigen des Hepatitis B-Virus kodiert, dadurch gekennzeichnet, dass das Fragment ein Oligo- oder Polypeptid gemäß einem der Gegenstände (1) bis (3) enthält.
(6) Isoliertes Oligo- oder Polynukleotid, welches für die a-Determinante des HBs-Antigens des Hepatitis B-Virus kodiert und ein Oligo- oder Polynukleotid gemäß einem der Gegenstände (1) bis (3) enthält.
(7) Primer, der für ein Oligo- oder Polynukleotid gemäß einem der Gegenstände (1) bis (6) spezifisch ist.
(8) Vektor, der mindestens ein Oligo- oder Polynukleotid gemäß einem der Gegenstände (1) bis (5) enthält.
(9) Wirtszelle, die einen Vektor gemäß (8) enthält.
(10) Oligo- oder Polypeptid welches durch ein Oligo- oder Polynukleotid gemäß einem der Gegenstände (1) bis (5) kodiert wird.
(11) Isoliertes Oligo- oder Polypeptid, das eine Aminosäuresequenz ausgewählt aus der Gruppe von Seq id no:12 bis Seq id no:22 hat: **Seq id no.: 17**
   **111** P G S T R T S T G Q 120 **Seq id no.: 19**
   **115**: R T S T G Q 120 **Seq id no.: 22**
   **154:** P I P L S W A F A K Y L W V W 165
(12) Oligo- oder Polypeptid gemäß (10) oder (11), das zu jeweils mindestens 65% oder 66% oder 67% oder 68% oder 69% oder 70% oder 71% oder 72% oder 73% oder 74% oder 75% oder 76% oder 77% oder 78% oder 79% oder 80% oder 81% oder 82% oder 83% oder 84% oder 85% oder 86% oder 87% oder 88% oder 89% oder 90% oder 91% oder 92% oder 93% oder 94% oder 95% oder 99% oder 97% oder 98% oder 99% mit einer der Sequenzen, ausgewählt aus der Gruppe von Seq id no:12 bis Seq id no:22, identisch ist.
(13) Isoliertes Polypeptid entsprechend der Sequenz des HBs-Antigens des Hepatitis B-Virus, dadurch gekennzeichnet, dass es ein Oligo- oder Polypeptid gemäß einem der Gegenstände (10) bis (12) enthält.
(14) Fragment eines Polypeptids, welches der Sequenz des HBs-Antigens des Hepatitis B-Virus entspricht, dadurch gekennzeichnet, dass das Fragment ein Oligo- oder Polypeptid gemäß einem der Gegenstände (10) bis (12) enthält.
(15) Isoliertes Polypeptid, welches für die a-Determinante des HBs-Antigens des Hepatitis B-Virus kodiert, dadurch gekennzeichnet, dass es ein Oligo- oder Polypeptid gemäß einem der Gegenstände (10) bis (12) enthält.
(16) Monoklonaler oder polyklonaler Antikörper, der an HBs-Antigen, enthaltend ein Oligo-oder Polypeptid gemäß einem der Gegenstände (10) bis (15) bindet, der an HBs-Antigen eines Hepatitis B-Wildtypvirus aber nicht oder wenigstens signifikant schwächer bindet.
(17) Ein anti-idiotypischer Antikörper, der eine Aminosäuresequenz gemäß einem der Gegenstände (10) bis (15) repräsentiert.
(18) Testkit zum Nachweis oder zur Bestimmung mittels einer Hybridisierungsreaktion einer Nukleinsäure, die für eine Variante oder Mutante des Hepatitis B-Virus spezifisch ist, unter Verwendung mindestens eines Oligo- oder Polynukleotids gemäß einem oder mehreren der Gegenstände (1) bis (7).
(19) Testkit zum immunchemischen Nachweis oder zur immunchemischen Bestimmung eines Antigens, das für eine Variante oder Mutante des Hepatitis B-Virus spezifisch ist, unter Verwendung mindestens eines monoklonalen oder polyklonalen Antikörpers gemäß (16).
(20) Testkit zum immunchemischen Nachweis oder zur immunchemischen Bestimmung eines gegen eine Variante oder Mutante des Hepatitis B-Virus gerichteten Antikörpers unter Verwendung mindestens eines Oligo- oder Polypeptids gemäß einem der Gegenstände (10) bis (15).
(21) Immunogenes Peptid oder Mischung immunogener Peptide enthaltend ein oder mehrere Oligo- oder Polypeptide gemäß einem oder mehreren der Gegenstände (3) und (4) alleine oder in Verbindung mit bekannten HBV-Immunogenen.

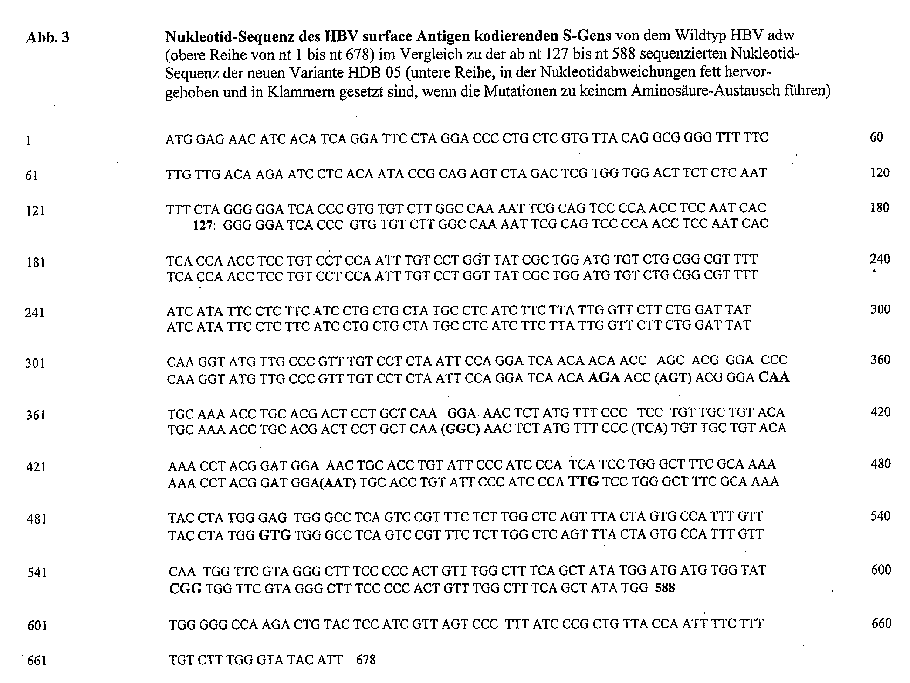

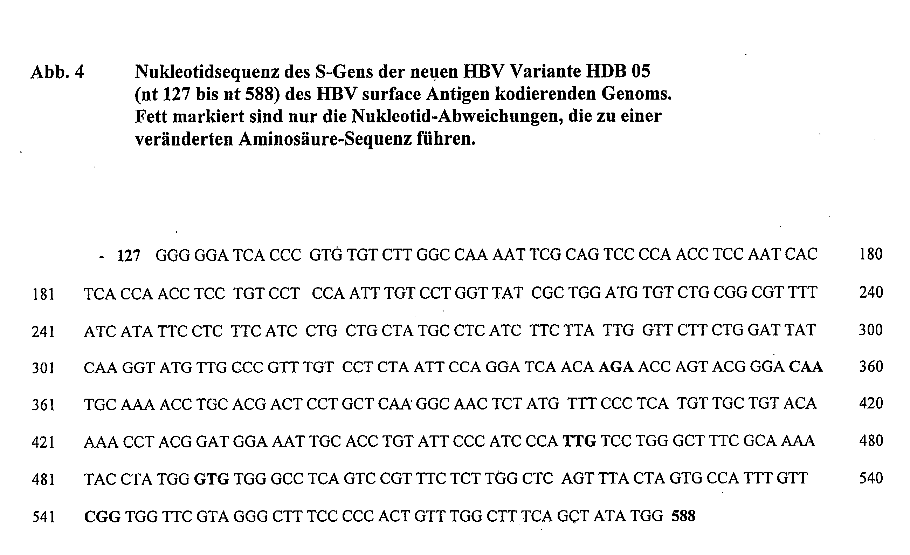

Die vorliegende Ofenbarung umfasst eine isolierte Nukleotid-Sequenz, die zu mindestens 65 % mit Seq id no. 1 identisch ist bzw. mit einem Fragment dieser in Abb. 3 und 4 dargestellten Sequenz, das spezifisch zum Komplement der SEQ ID NO:1 bis 11 hybridisiert.

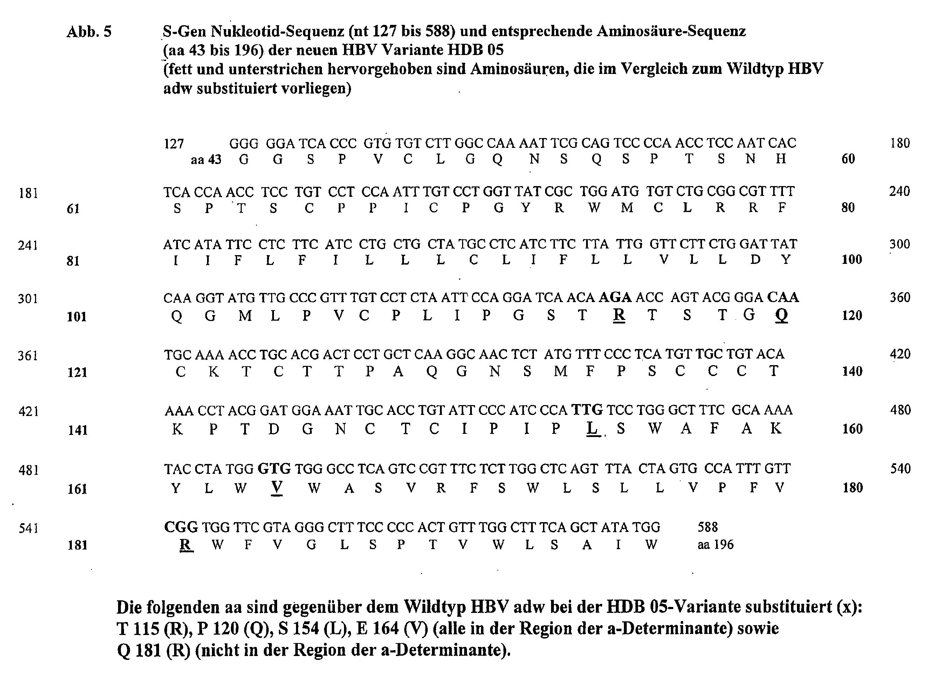

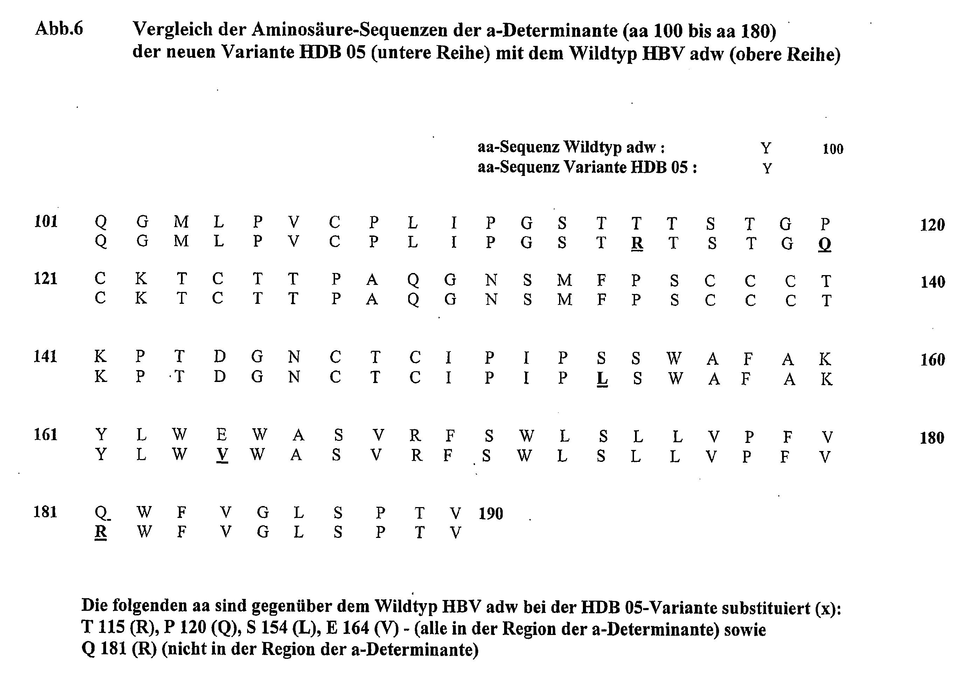

Zusätzlich beinhaltet die vorliegende Offenbarung eine isolierte Nukleotid-Sequenz, die die vorliegende erfindungsgemäße Variante der a-Determinante des Hepatitis B surface Antigens (HBsAg) in den Aminosäuren-Positionen zwischen aa 101 und 180 kodiert bzw. zu einem Peptid-Produkt führt, das in der aa-Sequenz zu mindestens 65 % mit der in Abb. 5 und 6 dargestellten SEQ ID NO:12 übereinstimmt oder Fragmenten davon gemäß SEQ ID NO:13 bis 22.

Des weiteren beeinhaltet die vorliegende Offenbarung einen Vektor, umfassend eine oder mehrere der genannten Nukleotid-Sequenzen wie auch eine Wirtszelle, die diesen Vektor enthält und eine Methode zur Darstellung eines entsprechenden Polypeptides aus der a-Determinante, umfassend die Inkubation der oben genannten Wirtszelle über Zeiten und unter Bedingungen, die für die Expression des Polypeptides erforderlich sind.

Auch Gegenstand der Ofenbarung sind Antikörper, die mit der in SEQ ID NO:11 bis 22 beschriebenen a-Determinante reagieren, wobei die Bindung bevorzugt in dem Aminosäurebereich aa 115 bis 120, aa 154 bis 164 oder aa 154 bis 185 erfolgt. Die Antikörper können polyklonalen oder monoklonalen tierischen oder menschlichen Ursprungs sein.

Eine isolierte HBV Variante ist ebenfalls Gegenstand der Offenbarung, wobei das Virus eine a-Determinante aufweist, die den aa-Sequenzen mindestens zwischen Position 115 und 120 und/oder aa 154 bis164 bzw. aa 154 bis 181 entspricht, idealerweise allen genannten Regionen zwischen 115 und 181.

Auch eine immunogene Mischung zur Erzeugung polyklonaler oder monoklonaler Antikörper ist Gegenstand der vorliegenden Ofenbarung umfassend das beschriebene isolierte HBV oder ein bzw. mehrere der beschriebenen Poypeptide.

Die Offenbarung beinhaltet auch eine Poly-Nukleotid-Sonde, enthaltend eine HBV Genom-Sequenz, welche durch Substitution von Aminosäuren zu einer modifizierten a-Determinanten führt, die mit der beschriebenen aa-Sequenz der neuen HBV-Variante identisch ist oder zu mindestens 65 % entspricht.

Auch Kits zum Nachweis von Polynukleotiden der HBV-Variante mit Hilfe der genannten Sonde wie auch Kits zum Nachweis von HBsAg der Variante bzw. einzelnen Epitopen davon und Antikörper, die für die Variante oder Epitope davon spezifisch sind, sind ebenso Gegenstand der Offenbarung, wie die Methoden des Nachweises von Polynukleotiden, Antigen und Antikörper, umfassend eine Inkubation zur Bildung entsprechender Komplexe und Nachweis dieser Komplexe durch geeignete dem Fachmann bekannte Verfahren.

Die Ausführungsformen dieser Kits und Nachweismethoden können zum spezifischen und alleinigen Nachweis von Nukleotiden und Antigen der HBV-Variante bzw. dagegen gerichteter Antikörper ausgelegt sein oder supplementär, d.h. den zusätzlichen Nachweis der erfindungsgemäßen Varianten-Analyten zu derzeitig bekannten HBV-Nukleotiden, - Antigenen bzw. - Antikörpern gestatten.

Analog kann eine immunogene Mischung von erfindungsgemäßen Polypeptidsequenzen auch in Verbindung mit bekannten Antigenen z.B. für die Verbesserung der Wirksamkeit einer Vakzine angewendet werden.

Die vorliegende Erfindung beschreibt eine neue Variante des Hepatitis B Virus (HBV), die eine völlig neue a-Determinante als Resultat von Aminosäure-Austauschen in den nachfolgenden aa-Positionen der S-HBsAg Sequenz aufweist. Für die Beschreibung der Aminosäuren wird der 1-Buchstaben-Code verwendet:

| aa von HDB 05 | aa-Position | aa von adw/Genotyp A |
|---|---|---|
| R | 115 | T |
| Q | 120 | P |
| L | 154 | S |
| V | 164 | E |

Außerdem liegt in Position aa 181 von HDB 05 Arginin (R) statt Gln (Q) vor:

| | | |
|---|---|---|
| R | 181 | Q. |

Diese aa-Substitutionen lassen sich auf entsprechende Nukleotid-Substitutionen der entsprechenden Codons zurückführen.

Die vorliegende Erfindung betrifft eine isolierte Nukleotid-Sequenz, die für die a-Determinante des Virus kodiert (Abb. 3 sowie Seq id no.: 1).

Die vorliegende Offenbarung umfasst auch Nukleotide mit mindestens 65 % Übereinstimmung, bevorzugt mindestens 75% Übereinstimmung und besonders bevorzugt mit mindestens 90% Übereinstimmung mit der Nukleotid-Sequenz der vorliegenden Erfindung bzw. Fragmenten davon sowie dazu komplementäre Sequenzen.

Die Ofenbarung umfasst auch Polypeptide, die von oben beschriebenen Nukleotid-Sequenzen kodiert werden, insbesondere solche Aminosäuresequenzen, die die a-Determinante des HBsAg bestimmen und Polypeptide, die mindestens eine Ähnlichkeit von 65%, bevorzugt 75 % und noch bevorzugter 95 % zu diesen Sequenzen aufweisen.

Für die Beschreibung der vorliegenden Erfindung wird unter einem Nukleotid-Fragment eine konsekutive Folge von mindestens 9, bevorzugt 9-15, besonders bevorzugt 15-21 und sogar ganz besonders bevorzugt 21-60 Nukleotide aus der Nukleotidsequenz der neuen HBV-Variante verstanden, wobei auch Mischungen derartiger Nukleotid-Fragmente naheliegen.

Ein Polypeptid-Fragment wird als Folge von mindestens 3, bevorzugt 3-5, besonders bevorzugt 5-7 und sogar ganz besonders bevorzugt 7-20 Aminosäuren aus der a-Determinante der neuen HBV-Variante verstanden, wobei auch Mischungen derartiger Polypeptid-Fragmente unter diese Erfindung fallen.

Unter die vorliegende Erfindung fällt auch eine isolierte Nukleotid-Sequenz, die hybridisierbar ist und zu Nukleotid-Sequenzen führt, die den Nukleotidsequenzen des HBsAg der neuen HBV Variante oder Teilen der a-Determinante der neuen HBV Variante entsprechen, komplementär dazu sind oder als Subtyp oder Mutation auf HDB 05 zurückzuführen sind.

Dem Fachmann ist bekannt, dass eine Nukleotidsequenz nach ihrer Isolierung nach Methoden gemäß Stand der Technik in prokaryontische (z.B. E. coli), eukaryontische Wirtszellen (z.B. Chinese Hamster Ovary Cell) oder Hefe (z.B. S. Cerevisiae) mit Hilfe eines Vektors oder Konstruktes eingebracht werden können (mit dem Fachmann bekannten Methoden wie z.B. Transfektion, Transformation oder Elektroporation: Molecular Cloning: A Laboratory Manual, 2nd ed., Vol. 1-3, ed Sambrook et al., Cold Spring Harbor Laboratory Press (1989), wobei transiente oder permanente Kulturen angewendet werden können. Demzufolge umfasst die vorliegende Erfindung isolierte Nukleotidsequenzen der a-Determinante der neuen HBV Variante, Polypeptide, die durch diese Nukleotide kodiert werden, Vektoren, die Nukleotidsequenzen der a-Determinante der neuen HBV Variante enthalten wie auch die Wirtszelle, in die ein Vektor gebracht wird.

Neben der Darstellung von Polypeptiden mit Hilfe eines Expressions-Systems (rekombinant oder gentechnologisch) ist es naheligend, dass analoge Polypeptid-Strukturen auch vollsynthetisch hergestellt werden oder direkt durch Reinigung aus der Virusvariante.

Es ist möglich, die Polypeptide oder Proteine der neuen HBV Variante zur Erzeugung von monoklonalen und/oder polyklonalen Antikörpern zu verwenden, die immunologisch an Bindestellen (Epitope) der a-Determinanteder neuen HBV Variante binden. Die Methoden zur Darstellung von Antikörpern sind dem Fachmann bekannt (z.B. Koehler et al., Nature 256-494 (1975), Mimms et al., Vi. 176: 604-619 (1990).

Ferner ist es möglich, die a-Determinante der erfindungsgemäßen HDB 05-Variante in Form der gesamten Polypeptidsequenz oder Teilen davon für die Bestimmung von gegen die HBV Variante gerichteten Antikörpern zu verwenden : Anti-HBs-Antikörper.

Dem Fachmann ist eine Vielzahl von Bestimmungsmethoden geläufig, bei denen mit Polypeptiden aus der a-Determinante der HBV Variante und Antikörpern tierischen oder menschlichen Ursprungs Immunkomplexe gebildet oder deren Bildung gehemmt werden.

Schließlich ist es möglich, monoklonale oder polyklonale Antikörper (oder Mischungen bzw. Fragmente davon oder Mischungen von Fragmenten), die mit Epitopen der neuen HBV Variante reagieren, dazu zu verwenden, die a-Determinante der erfindungsgemäßen HBV Variante in Form der gesamten Polypeptidsequenz oder Teilen davon in Untersuchungsproben zu bestimmen: HBsAg der HDB 05-Variante.

Dem Fachmann ist eine Vielzahl von Bestimmungsmethoden geläufig, bei denen mit einem oder mehreren monoklonalen Antikörper(n) oder polyklonalen Antikörpern (oder Mischungen davon bzw. Fragmenten oder Mischungen von Fragmenten), die spezifisch für die a-Determinante der HBV Variante sind, Immunkomplexe gebildet oder deren Bildung gehemmt werden.

Ebenso können auf Basis der gefundenen Nukleotidsequenzen der neuen HBV-Variante entsprechende Primer entwickelt werden.

Schließlich sind auch diagnostische Reagenzien als Kits Gegenstand der Erfindung, die basierend auf den oben beschriebenen Verfahren einen Nachweis von HBV Variantenspezifischem Antigen (HBsAg) oder dagegen gerichtete Antikörper (Anti-HBs), entweder als singuläre Bestimmungen oder miteinander oder mit anderen bekannten HBV-Antigen bzw. spezifisch damit reagierenden Antikörpern bzw. auch mit ganz anderen Analyten kombinierbar.

Die vorliegende Erfindung ist darüber hinaus in den Patentansprüchen beschrieben.

### Beschreibung der Abbildungen:

**Abb. 1** stellt eine Übersicht der Aminosäure- Sequenzen der a-Determinante von 6 beschriebenen Genotypen des HBV im Vergleich zu HDB 05 dar.
In **Abb. 2** sind die Nukleotid- und Aminosäure-Sequenzen der a-Determinante sowie unmittelbar benachbarter Regionen des Genotyps A, Subtyp adw von HBV dargestellt.
**Abb. 3** zeigt die Nuleotid-Sequenz der a-Determinante des HBV surface Antigens für Subtyp adw des Genotyps A von HBV im Vergleich zur Nukleotid-Sequenz von HDB 05.
**Abb. 4** fasst die Translations-relevanten Abweichungen der Nukleotid-Sequenz von HDB 05 zusammen.
In **Abb. 5** wird die Nukleotid-Sequenz von HDB 05 in der Region der a-Determinante sowie die entsprechende Aminosäure-Sequenz dargestellt. Die a-Determinante befindet sich zwischen Aminsäure No. 101 und 180 des kleinen HBsAg (Small, S).
**Abb. 6** zeigt die entsprechende Polypeptid-Sequenz der a-Determinante von HDB 05, die von der in Abb. 5 beschriebenen Nukleotid-Sequenz kodiert wird.

Die nachfolgenden Beispiele erläutern die vorliegende Erfindung näher, ohne dass die Erfindung auf die beschriebenen Beispiele beschränkt ist.

### Beispiel 1: HBsAg-Bestimmung mittels Enzymimmunoassay, EIA

Zur Bestimmung des surface Antigens von HBV, HBsAg im Blut der Patienten aus Frankreich und Österreich wurde der Enzymimmunoassay Enzygnost ® HBsAg 5.0 der Fa. Dade Behring GmbH, Marburg Deutschland angewendet.

Es handelt sich um einen in Europa zugelassenen und leistungsfähigen Test, der den Angaben der Packungsbeilage gemäß abgearbeitet wurde.

Das zu Grunde liegende Testprinzip ist ein sogenannter Sandwich Test im Mikrotiterplatten-Format:

100 µl der zu untersuchenden Probe werden in einem Einschritt-Verfahren mit 25 µl Konjugat 1 (monoklonale HBsAg spezifische Antikörper von der Maus, die kovalent mit Biotin markiert sind) und immobilisierten HBsAg-spezifischen polyklonalen Antikörpern vom Schaf in Kontakt gebracht. Nach 60-minütiger Inkubation bei 37°C und Entfernen überschüssiger Komponenten durch 4-maliges Waschen der Platten-Kavitäten werden 100 µl Konjugat 2 zugegeben, das aus Streptavidin besteht, an das das Sonden-Enzym Peroxidase kovalent gebunden ist.

Nach 30-minütiger Inkubation bei 37°C und Entfernen überschüssiger Komponenten durch 4-maliges Waschen der Platten-Kavitäten werden 75 µl Chromogen-Puffer/-Substrat-Lösung zugegeben, gefolgt von einer 30-minütigen Inkubation bei Raumtemperatur. Die Entwicklung des blau gefärbten Tetramethylbenzidin-Farbstoffes wird durch Zugabe von 75 µl Stoplösung (Schwefelsäure) unterbrochen und der Farbstoff bei 450 nm photometrisch gemessen.

Die Intensität der Farbentwicklung, gemessen an der optischen Dichte (O.D.) ist dem Gehalt der Untersuchungsprobe an HBsAg direkt proportional, wobei ein O.D. Wert von kleiner als dem Grenzwert als HBsAg-negativ bewertet wird. Der Grenzwert ist definiert als der O.D.-Mittelwert der parallel getesteten Kontrolle, negativ (im Testkit enthalten), zu dem ein konstanter Betrag von 0,05 O.D. addiert wird.

Die Nachweisgrenzen des zur Untersuchung herangezogenen Lots (# 32874) wurden mit den international akzeptierten Standardpräparationen des Paul-Ehrlich-Institutes, Langen Deutschland zu 0,012 ng ad-Subtyp/ ml bzw. 0,015 ng ay-Subtyp / ml in parallelen Versuchsansätzen aus Testungen von Verdünnungen der Standardpräparationen in HBsAgnegativem Serum durch graphische Interpolation ermittelt.

Die Untersuchung der Proben # 119617 und 118234, aus denen auch die DNA-Isolierung vorgenommen wurde, brachte in 2 unabhängigen Versuchen an zwei verschiedenen Tagen für beide Proben Ergebnisse zwischen 0,02 und 0,05 O.D., die den Kriterien des Testes entsprechend als HBsAg-negativ zu interpretieren sind. Die mitgeführte Kontrolle, positiv (im Testkit enthalten) war dagegen ebenso positiv (Validierungskriterien erfüllt) wie die erwähnten ad- und ay-Standardpräparationen.

### Beispiel 2: Isolierung der HDB 05- DNA aus Probe # 118234

Aus je einem 200 µl-Aliquot der französischen und österreichischen Proben wurde die DNA isoliert, indem der QIA amp® DNA Blood Mini Kit der Fa. Qiagen, Hilden Deutschland) angewendet wurde. Dabei wurden alle Verfahrensschritte wie in der Packungsbeilage beschrieben befolgt und die Elution in einem Volumen von je 50 µl vorgenommen.

### Beispiel 3: Polymerase Ketten Reaktion, PCR

### 3.1 HBV Primer

Die vier nachstehenden HBV Primer wurden verwendet:
Primer 1 mit der 5'> 3'- Sequenz:
   GGGTCACCATATTCTTGGGAAC (SEQ ID NO:23)
Primer 2 mit der 5'> 3'- Sequenz:
   TATACCCAAAGACAAAAGAAAATTGG (SEQ ID NO:24)
Primer 3 mit der 5'> 3'- Sequenz:
   GACTCGTGGTGGACTTCTCTC (SEQ ID NO:25)
Primer 4 mit der 5'> 3'- Sequenz:
   TACAGACTTGGCCCCCAATACC (SEQ ID NO:26)

### 3.2 PCR-Amplifikation

Es wurde eine sogenannte nested PCR amplification des surface Antigens durchgeführt, wobei der Perkin Elmer Ampli Taq ® DNA Polymerase Kit sowie der Thermocycler Gene Amp ® PCR system 9700 der Fa. Perkin Elmer Applied Biosystems, USA verwendet wurde.

Die Nukleotide wurden von der Fa. Amersham Biosciences, UK bezogen.

Für den ersten Amplifikations-Zyklus wurden 5 µl der isolierten DNA unter Verwendung der oben genannten Primer 1 und 2 sowie folgenden Bedingungen amplifiziert:

### PCR 1 rxn

| | | | | |
|---|---|---|---|---|
| Primer 1 (10 µM) | | | 1 µl | |
| Primer 2 (10 µM) | | | 1 µl | |
| 10fach konz. Puffer (incl. 15 µM Mg2Cl) | | | 5 µl | |
| dNTP Mischung (10 µM) | | | 1 µl | |
| dest. Wasser | | | 36,75 µl | |
| Ampli Taq (5 U/ µl) | | | 0,25 µl | |
| | Pro Röhrchen | | 45 µl | Gesamtvolumen |
| | | plus | 5 µl | isolierte DNA |
| | | | 50 µl | Reaktionsvolumen |

Der 50 µl-Ansatz wurde unter Verwendung des beschriebenen Thermocyclers unter folgenden Bedingungen amplifiziert:
94° C, 1 min. / 94°C, 28 sek. - 50° C, 28 sek. - 72 ° C, 38 sek.
(35 cycles) / 72° C, 5 min. / 8 °C soak.

In der zweiten Amplifizierungsrunde wurde 5 µl des ersten PCR Produktes weiter amplifiziert unter Verwendung der HBV Primer 3 und 4 und folgenden Bedingungen:

### PCR 2 rxn

| | | | | |
|---|---|---|---|---|
| Primer 3 (10 µM) | | | 1 µl | |
| Primer 4 (10 µM) | | | 1 µl | |
| 10 -fach konz. Puffer | | | 5 µl | |
| dNTP Mischung (10 µM) | | | 1 µl | |
| dest. Wasser | | | 36,75 µl | |
| Ampli Taq (5 U/µl) | | | 0,25 µl | |
| | Pro Röhrchen | | 45 µl | Gesamtvolumen |
| | | plus | 5 µl | PCR Produkt v.rxn |
| | | | 50 µl | Reaktionsvolumen |

Dieser PCR 2- Ansatz wurde unter Verwendung des oben beschriebenen Thermocyclers amplifiziert wobei folgende Bedingungen angewendet wurden:
94° C, 1 min. / 94°C, 28 sek. - 55° C, 28 sek. - 72° C, 38 sek.
(35 cycles) / 72 °C, 5 min. / 8 °C soak.

Abschließend wurde das PCR 2 Produkt elektrophoretisch aufgetrennt (1,5 % Agarose) unter Mitführen geeigneter Molekulargewichtsmarker. Die Bande mit ca. 520 Basenpaaren wurde ausgeschnitten und mit Hilfe des QIA quick Gel Extraction Kit der Fa. Qiagen, Hilden Deutschland isoliert .

### Beispiel 4: Sequenzierung von HDB 05

Das gereinigte PCR Produkt wurde von der Fa. Medigenomix, Martinsried Deutschland mit Hilfe des ABI 3700 Kapillar Systems sequenziert in Verbindung mit der ABI BigDye Terminator Chemistry Version 1.1. und der ABI Sequencing Analysis Software Version 3.6. unter Verwendung der in Bsp. 3 beschriebenen Primer 3 und 4.

### Sequenzierungs-Ergebnis

Es konnte gezeigt werden, dass das HBsAg beider analysierten Proben miteinander übereinstimmt und innerhalb des sequenzierten Bereiches die beste

Übereinstimmung der Nukleotid- und Aminosäure-Sequenz mit dem Genoty A, Subtyp adw zeigt. In der Region der a-Determinante zeigten die analysierten Proben aus Frankeich und Österreich miteinander übereinstimmend insgesamt 4 Aminosäure-Substitutionen im Vergleich zum Genotyp A, Subtyp adw (siehe auch Abb. 2 und 5):

| **HDB 05:** | | | **A, adw:** |
|---|---|---|---|
| 1.) | Arg (R) | gegen | 115 Thr (T) |
| 2.) | Gln (Q) | gegen | 120 Pro (P) |
| 3.) | Leu (L) | gegen | 154 Ser (S) |
| 4.) | Val (V) | gegen | 164 Glu (E) |

Zusätzlich liegt eine Aminosäure-Substitution in der Position # 181 vor:

| | | | |
|---|---|---|---|
| 5.) | Arg (R) | gegen | 181 Gln (Q). |

Diese Ergebnisse wurden in mehreren unabhängigen Analysen beider untersuchter Blutproben aus Frankreich und Österreich mit den gleichen Sequenzierungs-Ergebnissen reproduziert, die überdies für beide unabhängige Proben völlige Übereinstimmung aufweisen.

## Patentansprüche

1. Oligo- oder Polypeptid umfassend
(a) eine Aminosäuresequenz, die eine Teilsequenz von SEQ ID NO:12 mit wenigstens 70 aufeinanderfolgenden Aminosäuren von SEQ ID NO:12 ist, wobei die Teilsequenz die Positionen 73, 78, 112, 122 und 139 von SEQ ID NO:12 einschließt; oder
(b) ein Fragment eines HBs-Antigens eines Hepatitis B-Virus gemäß Abbildung 2, wobei das HBs-Antigen an Position 115 Arginin, an Position 120 Glutamin, an Position 154 Leucin, an Position 164 Valin und an Position 181 Arginin aufweist und das Fragment Arginin 115, Glutamin 120, Leucin 154, Valin 164 und Arginin 181 umfasst.

2. Oligo- oder Polypeptid nach Anspruch 1, **dadurch gekennzeichnet, dass** es eine Aminosäuresequenz umfasst, die ausgewählt ist aus der Gruppe bestehend aus SEQ ID NO:12 und SEQ ID NO:13.

3. Oligo- oder Polynukleotid umfassend
(a) eine Nukleotidsequenz, die eine Teilsequenz von SEQ ID NO:1 mit wenigstens 200 aufeinanderfolgenden Nukleotiden von SEQ ID NO:1 ist, wobei die Teilsequenz die Positionen 218, 233, 335, 365 und 416 von SEQ ID NO:1 einschließt,
(b) eine Nukleotidsequenz, die unter stringenten Bedingungen spezifisch mit einem zu der Sequenz SEQ ID NO:1 komplementären Polynukleotid hybridisiert, oder
(c) eine Nukleotidsequenz, die für ein Oligo- oder Polypeptid nach einem der Ansprüche 1 bis 2 kodiert;
oder ein dazu komplementäres Oligo- oder Polynukleotid.

4. Oligo- oder Polynukleotid nach Anspruch 3, **dadurch gekennzeichnet, dass** es eine Nukleotidsequenz umfasst, die ausgewählt ist aus der Gruppe bestehend aus SEQ ID NO:1 oder SEQ ID NO:2.

5. Vektor oder Plasmid enthaltend ein Oligo- oder Polynukleotid nach einem der Ansprüche 3 oder 4.

6. Zelle, die mit einem Vektor oder Plasmid nach Anspruch 5 transformiert oder transfiziert wurde.

7. Zelle, die ein Oligo- oder Polynukleotid nach einem der Ansprüche 3 bis 4 oder einen Vektor oder ein Plasmid nach Anspruch 6 enthält.

8. Verfahren zur Herstellung eines Oligo- oder Polypeptids nach einem der Ansprüche 1 oder 2, das umfasst, dass man eine Zelle nach Anspruch 6 oder 7 unter geeigneten Bedingungen kultiviert, so dass das Oligo- oder Polypeptid exprimiert wird.

9. Verfahren nach Anspruch 8, **dadurch gekennzeichnet, dass** das Oligo- oder Polypeptid aus den Zellen gewonnen wird und von anderen Oligo- oder Polypeptiden abgetrennt wird.

10. Anti-idiotypischer Antikörper, der eine Aminosäuresequenz repräsentiert, wie sie in einem der Ansprüche 1 oder 2 definiert ist.

11. Testkit zum Nachweis von Hepatitis B-Viren, enthaltend
(i) ein Oligo- oder Polypeptid nach einem der Ansprüche 1 oder 2; oder
(ii) ein Oligo- oder Polynukleotid nach einem der Ansprüche 3 oder 4.

12. Immunogenes Peptid oder Mischung immunogener Peptide, enthaltend ein oder mehrere Oligo- oder Polypeptide nach einem der Ansprüche 1 oder 2 alleine oder in Verbindung mit bekannten HBV-Immunogenen.

13. Verfahren zum Nachweis von Antikörpern, die gegen ein Hepatitis B-Antigen gerichtet sind, **dadurch gekennzeichnet, dass**
(a) eine Probe mit einem Oligo- oder Polypeptid nach einem der Ansprüche 1 oder 2 unter Bedingungen inkubiert wird, die die Bildung eines Antigen-Antikörper-Komplexes gestatten; und
(b) der Antikörper-Antigen-Komplex, der das Oligo- oder Polypeptid enthält, nachgewiesen wird.

14. Verfahren zum Nachweis einer Hepatitis B-Nukleinsäure, **dadurch gekennzeichnet, dass**
(a) eine Probe mit einem Oligo- oder Polynukleotid nach einem der Ansprüche 3 oder 4 unter Bedingungen inkubiert wird, die die selektive Hybridisierung des Oligo- oder Polynukleotids mit einer Hepatitis B-Nukleinsäure in der Probe gestatten; und
(b) bestimmt wird, ob Polynukleotidduplexe gebildet wurden, die das Oligo- oder Polynukleotid umfassen.

15. Verfahren zum Nachweis einer Hepatitis B-Nukleinsäure, **dadurch gekennzeichnet, dass**
(a) eine Probe mit wenigstens einem Oligo- oder Polynukleotid nach einem der Ansprüche 3 oder 4 unter Bedingungen inkubiert wird, die die selektive Hybridisierung des Oligo- oder Polynukleotids mit einer Hepatitis B-Nukleinsäure in der Probe gestatten;
(b) eine Polymerasekettenreaktion durchgeführt wird; und
(c) bestimmt wird, ob eine Nukleinsäure amplifiziert wurde.

16. Verwendung eines Oligo- oder Polynukleotids nach einem der Ansprüche 3 bis 5 als Primer.

17. Verwendung eines Oligo- oder Polynukleotids nach einem der Ansprüche 3 oder 4 als Sonde.

18. Isoliertes Hepatitis B-Virus, das eine a-Determinante aufweist die der Aminosäuresequenz mindestens der Positionen 115 bis 181 gemäß Anspruch 1(b) entspricht.

## Claims

1. Oligopeptide or polypeptide comprising
(a) an amino acid sequence which is a constituent sequence of SEQ ID NO: 12 containing at least 70 consecutive amino acids of SEQ ID NO: 12, with the constituent sequence including the positions 73, 78, 112, 122 and 139 of SEQ ID NO: 12; or
(b) a fragment of an HBs antigen of a hepatitis B virus according to Figure 2, with the HBs antigen possessing arginine at position 115, glutamine at position 120, leucine at position 154, valine at position 164 and arginine at position 181 and the fragment comprising arginine 115, glutamine 120, leucine 154, valine 164 and arginine 181.

2. Oligopeptide or polypeptide according to Claim 1, **characterized in that** it comprises an amino acid sequence which is selected from the group consisting of SEQ ID NO: 12 and SEQ ID NO: 13.

3. Oligonucleotide or polynucleotide comprising
(a) a nucleotide sequence which is a constituent sequence of SEQ ID NO: 1 containing at least 200 consecutive nucleotides of SEQ ID NO: 1, with the constituent sequence including the positions 218, 233, 335, 365 and 416 of SEQ ID NO: 1,
(b) a nucleotide sequence which specifically hybridizes, under stringent conditions, with a polynucleotide which is complementary to the sequence SEQ ID NO: 1, or
(c) a nucleotide sequence which encodes an oligopeptide or polypeptide according to either of Claims 1 and 2;
or an oligonucleotide or polynucleotide which is complementary thereto.

4. Oligonucleotide or polynucleotide according to Claim 3, **characterized in that** it comprises a nucleotide sequence which is selected from the group consisting of SEQ ID NO: 1 or SEQ ID NO: 2.

5. Vector or plasmid which contains an oligonucleotide or polynucleotide according to either of Claims 3 or 4.

6. Cell which has been transformed or transfected with a vector or plasmid according to Claim 5.

7. Cell which contains an oligonucleotide or polynucleotide according to either of Claims 3 and 4 or a vector or plasmid according to Claim 6.

8. Method for preparing an oligopeptide or polypeptide according to either of Claims 1 or 2 which comprises culturing a cell according to Claim 6 or 7 under suitable conditions such that the oligopeptide or polypeptide is expressed.

9. Method according to Claim 8, **characterized in that** the oligopeptide or polypeptide is isolated from the cells and separated off from other oligopeptides or polypeptides.

10. Antiidiotypic antibody which represents an amino acid sequence as defined in either of Claims 1 or 2.

11. Test kit for detecting hepatitis B viruses, comprising
(i) an oligopeptide or polypeptide according to either of Claims 1 or 2; or
(ii) an oligonucleotide or polynucleotide according to either of Claims 3 or 4.

12. Immunogenic peptide or mixture of immunogenic peptides containing one or more oligopeptides or polypeptides according to either of Claims 1 or 2 on its/their own or in combination with known HBV immunogens.

13. Method for detecting antibodies which are directed against a hepatitis B antigen, **characterized in that**
(a) a sample is incubated with an oligopeptide or polypeptide according to either of Claims 1 or 2 under conditions which allow the formation of an antigen-antibody complex; and
(b) the antibody-antigen complex which contains the oligopeptide or polypeptide is detected.

14. Method for detecting a hepatitis B nucleic acid, **characterized in that**
(a) a sample is incubated with an oligonucleotide or polynucleotide according to either of Claims 3 or 4 under conditions which allow the selective hybridization of the oligonucleotide or polynucleotide with a hepatitis B nucleic acid in the sample; and
(b) it is determined whether polynucleotide duplexes which comprise the oligonucleotide or polynucleotide have been formed.

15. Method for detecting a hepatitis B nucleic acid, **characterized in that**
(a) a sample is incubated with at least one oligonucleotide or polynucleotide according to either of Claims 3 or 4 under conditions which allow the selective hybridization of the oligonucleotide or polynucleotide with a hepatitis B nucleic acid in the sample;
(b) a polymerase chain reaction is carried out; and
(c) it is determined whether a nucleic acid has been amplified.

16. Use of an oligonucleotide or polynucleotide according to one of Claims 3 to 5 as a primer.

17. Use of an oligonucleotide or polynucleotide according to either of Claims 3 or 4 as a probe.

18. Isolated hepatitis B virus which possesses a determinant which corresponds to the amino acid sequence of at least positions 115 to 181 according to Claim 1(b).

## Revendications

1. Oligopeptide ou polypeptide comprenant
(a) une séquence d'acides aminés, qui est une séquence partielle de l'identification de séquence N°12, ayant au moins 70 acides aminés successifs de l'identification de séquence N°12, la séquence partielle incluant les positions 73, 78, 112, 122 et 139 de l'identification de séquence N°12 ; ou
(b) un fragment d'un antigène d'HBs d'un virus de l'hépatite B suivant la Figure 2, dans lequel l'antigène d'HBs a en position 115 de l'arginine, en position 120 de la glutamine, en position 154 de la leucine, en position 164 de la valine et en position 181 de l'arginine, et le fragment comprend de l'arginine 115, de la glutamine 120, de la leucine 154, de la valine 164 et de l'arginine 181.

2. Oligopeptide ou polypeptide suivant la revendication 1, **caractérisé en ce qu'**il comprend une séquence d'acides aminés, qui est choisie dans le groupe constitué de l'identification de séquence N°12 et de l'identification de séquence N°13.

3. Oligonucléotide ou polynucléotide comprenant
(a) une séquence de nucléotides, qui est une séquence partielle de l'identification de séquence N°1, ayant au moins 200 nucléotides successifs de l'identification de séquence N°1, la séquence partielle incluant les positions 218, 233, 335, 365 et 416 de l'identification de séquence N°1,
(b) une séquence de nucléotides, qui s'hydride dans des conditions sévères spécifiquement avec un polynucléotide complémentaire de la séquence de l'identification de séquence N°1, ou
(c) une séquence de nucléotides, qui code pour un oligopeptide ou un polypeptide suivant l'une des revendications 1 à 2 ;
ou un oligonucléotide ou un polynucléotide qui en est complémentaire.

4. Oligonucléotide ou polynucléotide suivant la revendication 3, **caractérisé en ce qu'**il comprend une séquence de nucléotides, qui est choisie dans le groupe constitué de l'identification de séquence N°1 ou de l'identification de séquence N°2.

5. Vecteur ou plasmide contenant un oligonucléotide ou un polynucléotide suivant l'une des revendications 3 ou 4.

6. Cellule qui a été transformée ou transfectée par un vecteur ou par un plasmide suivant la revendication 5.

7. Cellule qui contient un oligonucléotide ou un polynucléotide suivant l'une des revendications 3 ou 4 ou un vecteur ou un plasmide suivant la revendication 6.

8. Procédé de préparation d'un oligopeptide ou d'un polypeptide suivant l'une des revendications 1 ou 2, dans lequel on cultive une cellule suivant la revendication 6 ou 7 dans des conditions appropriées, de manière à exprimer l'oligopeptide ou le polypeptide.

9. Procédé suivant la revendication 8, **caractérisé en ce que** l'on recueille l'oligopeptide ou le polypeptide à partir des cellules et on le sépare d'autres oligopeptides ou d'autres polypeptides.

10. Anticorps anti-idiotypique, qui représente une séquence d'acides aminés, telle qu'elle est définie dans l'une des revendications 1 ou 2.

11. Trousse de test pour la détection de virus de l'hépatite B, contenant
(I) un oligopeptide ou un polypeptide suivant l'une des revendications 1 ou 2 ; ou
(II) un oligonucléotide ou un polynucléotide suivant l'une des revendications 3 ou 4.

12. Peptide immunogène ou mélange de peptides immunogènes, contenant un ou plusieurs oligopeptides ou polypeptides suivant l'une des revendications 1 ou 2, seuls ou en liaison avec des immunogènes d'HBV connus.

13. Procédé de détection d'anticorps, qui sont dirigés contre un antigène de l'hépatite B, **caractérisé en ce que**
(a) on fait incuber un échantillon ayant un oligopeptide ou un polypeptide suivant l'une des revendications 1 ou 2, dans des conditions qui autorisent la formation d'un complexe antigène-anticorps ; et
(b) on détecte le complexe anticorps-antigène qui contient l'oligopeptide ou le polypeptide.

14. Procédé de détection d'un acide nucléique de l'hépatite B, **caractérisé en ce que**
(a) on fait incuber un échantillon comprenant un oligonucléotide ou un polynucléotide suivant l'une des revendications 3 ou 4, dans des conditions qui autorisent l'hybridation sélective de l'oligonucléotide ou du polynucléotide avec un acide nucléique de l'hépatite B de l'échantillon ; et
(b) on détermine si se sont formés des duplex de polynucléotide, qui comprennent l'oligonucléotide ou le polynucléotide.

15. Procédé de détection d'un acide nucléique de l'hépatite B, **caractérisé en ce que**
(a) on fait incuber un échantillon comprenant au moins un oligonucléotide ou un polynucléotide suivant l'une des revendications 3 ou 4, dans des conditions qui autorisent l'hybridation sélective de l'oligonucléotide ou du polynucléotide avec un acide nucléique de l'hépatite B de l'échantillon ;
(b) on effectue une réaction en chaîne de la polymérase ; et
(c) on détermine si un acide nucléique a été amplifié.

16. Utilisation d'un oligonucléotide ou d'un polynucléotide suivant l'une des revendications 3 à 5 comme amorce.

17. Utilisation d'un oligonucléotide ou d'un polynucléotide suivant l'une des revendications 3 à 4 comme sonde.

18. Virus de l'hépatite B isolé, qui comporte un déterminant a, qui correspond à la séquence d'acides aminés, au moins en des positions 115 à 181 suivant la revendication 1 (b).


### SEQUENCE LISTING

<110> Dade Behring Marburg GmbH
<120> Neue Oberflaechenprotein- (HBsAg-) Variante des Hepatitis B Virus
<130> MA 1252
<150> DE 10328080.4
   <151> 2003-06-20
<160> 26
<170> PatentIn version 3.2
<210> 1
   <211> 462
   <212> DNA
   <213> Hepatitis B virus
<400> 1
   gggggatcac ccgtgtgtct tggccaaaat tcgcagtccc caacctccaa tcactcacca      60
   acctcctgtc ctccaatttg tcctggttat cgctggatgt gtctgcggcg ttttatcata     120
   ttcctcttca tcctgctgct atgcctcatc ttcttattgg ttcttctgga ttatcaaggt     180
   atgttgcccg tttgtcctct aattccagga tcaacaagaa ccagtacggg acaatgcaaa     240
   acctgcacga ctcctgctca aggcaactct atgtttccct catgttgctg tacaaaacct     300
   acggatggaa attgcacctg tattcccatc ccattgtcct gggctttcgc aaaataccta     360
   tgggtgtggg cctcagtccg tttctcttgg ctcagtttac tagtgccatt tgttcggtgg     420
   ttcgtagggc tttcccccac tgtttggctt tcagctatat gg                        462
<210> 2
   <211> 225
   <212> DNA
   <213> Hepatitis B virus
<400> 2
   ccaggatcaa caagaaccag tacgggacaa tgcaaaacct gcacgactcc tgctcaaggc 60
   aactctatgt ttccctcatg ttgctgtaca aaacctacgg atggaaattg cacctgtatt 120
   cccatcccat tgtcctgggc tttcgcaaaa tacctatggg tgtgggcctc agtccgtttc 180
   tcttggctca gtttactagt gccatttgtt cggtggttcg taggg 225
<210> 3
   <211> 180
   <212> DNA
   <213> Hepatitis B virus
<400> 3
   ccaggatcaa caagaaccag tacgggacaa tgcaaaacct gcacgactcc tgctcaaggc 60
   aactctatgt ttccctcatg ttgctgtaca aaacctacgg atggaaattg cacctgtatt 120
   cccatcccat tgtcctgggc tttcgcaaaa tacctatggg tgtgggcctc agtccgtttc 180
<210> 4
   <211> 165
   <212> DNA
   <213> Hepatitis B virus
<400> 4
   ccaggatcaa caagaaccag tacgggacaa tgcaaaacct gcacgactcc tgctcaaggc 60
   aactctatgt ttccctcatg ttgctgtaca aaacctacgg atggaaattg cacctgtatt 120
   cccatcccat tgtcctgggc tttcgcaaaa tacctatggg tgtgg 165
<210> 5
   <211> 60
   <212> DNA
   <213> Hepatitis B virus
<400> 5
   ccaggatcaa caagaaccag tacgggacaa tgcaaaacct gcacgactcc tgctcaaggc 60
<210> 6
   <211> 30
   <212> DNA
   <213> Hepatitis B virus
<400> 6
   ccaggatcaa caagaaccag tacgggacaa 30
<210> 7
   <211> 153
   <212> DNA
   <213> Hepatitis B virus
<400> 7
   agaaccagta cgggacaatg caaaacctgc acgactcctg ctcaaggcaa ctctatgttt 60
   ccctcatgtt gctgtacaaa acctacggat ggaaattgca cctgtattcc catcccattg 120
   tcctgggctt tcgcaaaata cctatgggtg tgg 153
<210> 8
   <211> 18
   <212> DNA
   <213> Hepatitis B virus
<400> 8
   agaaccagta cgggacaa 18
<210> 9
   <211> 96
   <212> DNA
   <213> Hepatitis B virus
<400> 9
   ttgtcctggg ctttcgcaaa atacctatgg gtgtgggcct cagtccgttt ctcttggctc 60
   agtttactag tgccatttgt tcggtggttc gtaggg 96
<210> 10
   <211> 51
   <212> DNA
   <213> Hepatitis B virus
<400> 10
   ttgtcctggg ctttcgcaaa atacctatgg gtgtgggcct cagtccgttt c 51
<210> 11
   <211> 36
   <212> DNA
   <213> Hepatitis B virus
<400> 11
   ttgtcctggg ctttcgcaaa atacctatgg gtgtgg 36
<210> 12
   <211> 154
   <212> PRT
   <213> Hepatitis B virus
<400> 12
   Gly Gly Ser Pro Val Cys Leu Gly Gln Asn Ser Gln Ser Pro Thr Ser
   1               5                   10                  15
   Asn His Ser Pro Thr Ser Cys Pro Pro Ile Cys Pro Gly Tyr Arg Trp
               20                  25                  30
   Met Cys Leu Arg Arg Phe Ile Ile Phe Leu Phe Ile Leu Leu Leu Cys
           35                  40                  45
   Leu Ile Phe Leu Leu Val Leu Leu Asp Tyr Gln Gly Met Leu Pro Val
       50                  55                  60
   Cys Pro Leu Ile Pro Gly Ser Thr Arg Thr Ser Thr Gly Gln Cys Lys
   65                  70                  75                  80
   Thr Cys Thr Thr Pro Ala Gln Gly Asn Ser Met Phe Pro Ser Cys Cys
                   85                  90                  95
   Cys Thr Lys Pro Thr Asp Gly Asn Cys Thr Cys Ile Pro Ile Pro Leu
               100                 105                 110
   Ser Trp Ala Phe Ala Lys Tyr Leu Trp Val Trp Ala Ser Val Arg Phe
           115                 120                 125
   Ser Trp Leu Ser Leu Leu Val Pro Phe Val Arg Trp Phe Val Gly Leu
       130                 135                 140
   Ser Pro Thr Val Trp Leu Ser Ala Ile Trp
   145                 150
<210> 13
   <211> 75
   <212> PRT
   <213> Hepatitis B virus
<400> 13
   Pro Gly Ser Thr Arg Thr Ser Thr Gly Gln Cys Lys Thr Cys Thr Thr
   1               5                   10                  15
   Pro Ala Gln Gly Asn Ser Met Phe Pro Ser Cys Cys Cys Thr Lys Pro
               20                  25                  30
   Thr Asp Gly Asn Cys Thr Cys Ile Pro Ile Pro Leu Ser Trp Ala Phe
           35                  40                  45
   Ala Lys Tyr Leu Trp Val Trp Ala Ser Val Arg Phe Ser Trp Leu Ser
       50                  55                  60
   Leu Leu Val Pro Phe Val Arg Trp Phe Val Gly
   65                  70                  75
<210> 14
   <211> 60
   <212> PRT
   <213> Hepatitis B virus
<400> 14
   Pro Gly Ser Thr Arg Thr Ser Thr Gly Gln Cys Lys Thr Cys Thr Thr
   1               5                   10                  15
   Pro Ala Gln Gly Asn Ser Met Phe Pro Ser Cys Cys Cys Thr Lys Pro
               20                  25                  30
   Thr Asp Gly Asn Cys Thr Cys Ile Pro Ile Pro Leu Ser Trp Ala Phe
           35                  40                  45
   Ala Lys Tyr Leu Trp Val Trp Ala Ser Val Arg Phe
       50                  55                  60
<210> 15
   <211> 55
   <212> PRT
   <213> Hepatitis B virus
<400> 15
   Pro Gly Ser Thr Arg Thr Ser Thr Gly Gln Cys Lys Thr Cys Thr Thr
   1               5                   10                  15
   Pro Ala Gln Gly Asn Ser Met Phe Pro Ser Cys Cys Cys Thr Lys Pro
               20                  25                  30
   Thr Asp Gly Asn Cys Thr Cys Ile Pro Ile Pro Leu Ser Trp Ala Phe
           35                  40                  45
   Ala Lys Tyr Leu Trp Val Trp
       50                  55
<210> 16
   <211> 20
   <212> PRT
   <213> Hepatitis B virus
<400> 16
   Pro Gly Ser Thr Arg Thr Ser Thr Gly Gln Cys Lys Thr Cys Thr Thr
   1               5                   10                  15
   Pro Ala Gln Gly
               20
<210> 17
   <211> 10
   <212> PRT
   <213> Hepatitis B virus
<400> 17
   Pro Gly Ser Thr Arg Thr Ser Thr Gly Gln
   1               5                   10
<210> 18
   <211> 51
   <212> PRT
   <213> Hepatitis B virus
<400> 18
   Arg Thr Ser Thr Gly Gln Cys Lys Thr Cys Thr Thr Pro Ala Gln Gly
   1               5                   10                  15
   Asn Ser Met Phe Pro Ser Cys Cys Cys Thr Lys Pro Thr Asp Gly Asn
               20                  25                  30
   Cys Thr Cys Ile Pro Ile Pro Leu Ser Trp Ala Phe Ala Lys Tyr Leu
           35                  40                  45
   Trp Val Trp
       50
<210> 19
   <211> 6
   <212> PRT
   <213> Hepatitis B virus
<400> 19
   Arg Thr Ser Thr Gly Gln
   1               5
<210> 20
   <211> 36
   <212> PRT
   <213> Hepatitis B virus
<400> 20
   Pro Ile Pro Leu Ser Trp Ala Phe Ala Lys Tyr Leu Trp Val Trp Ala
   1               5                   10                  15
   Ser Val Arg Phe Ser Trp Leu Ser Leu Leu Val Pro Phe Val Arg Trp
               20                  25                  30
   Phe Val Gly Leu
           35
<210> 21
   <211> 20
   <212> PRT
   <213> Hepatitis B virus
<400> 21
   Pro Ile Pro Leu Ser Trp Ala Phe Ala Lys Tyr Leu Trp Val Trp Ala
   1 5 10 15
   Ser Val Arg Phe
   20
<210> 22
   <211> 15
   <212> PRT
   <213> Hepatitis B virus
<400> 22
   Pro Ile Pro Leu Ser Trp Ala Phe Ala Lys Tyr Leu Trp Val Trp
   1 5 10 15
<210> 23
   <211> 22
   <212> DNA
   <213> Artificial Sequence
<220>
   <223> Primer 1
<400> 23
   gggtcaccat attcttggga ac 22
<210> 24
   <211> 26
   <212> DNA
   <213> Artificial Sequence
<220>
   <223> Primer 2
<400> 24
   tatacccaaa gacaaaagaa aattgg 26
<210> 25
   <211> 21
   <212> DNA
   <213> Artificial Sequence
<220>
   <223> Primer 3
<400> 25
   gactcgtggt ggacttctct c 21
<210> 26
   <211> 22
   <212> DNA
   <213> Artificial Sequence
<220>
   <223> Primer 4
<400> 26
   tacagacttg gcccccaata cc 22